# EUROPEAN PATENT APPLICATION

(11) **EP 4 623 931 A1**
(43) Date of publication of application: **01.10.2025**
(21) Application number: 23894639.6
(22) Date of filing: 22.11.2023
(51) Int. Cl.: A61K 39/395, A61K 35/15, A61K 35/17, A61P 35/02, C07K 14/705, C07K 16/28, C07K 19/00, C12N 5/10, C12N 5/078, C12N 15/13, C12N 15/62

(54) **ANTIBODY AGAINST HEMATOLOGICAL CANCER**

(30) Priority: 25.11.2022 JP 2022188561
(71) Applicant: The University of Osaka, Suita-shi, Osaka 565-0871 (JP)
(72) Inventor: HOSEN, Naoki, Suita-shi, Osaka 565-0871 (JP)
(74) Representative: Zwicker, Jörk
(86) International application number: PCT/JP2023/042029
(87) International publication number: WO 2024/111635

(57) **Abstract**

An antibody that recognizes HLA-DR or a functional fragment thereof, a chimeric antigen receptor that has the antibody or a functional fragment thereof, an immune cell expressing the chimeric antigen receptor, a multispecific antibody that includes the antibody or a functional fragment thereof and the antigen recognition site for immune cells, or a pharmaceutical composition containing these antibodies and the like.

## Description

### [Technical Field]

The present invention relates to an antibody against a blood cancer, and more specifically pertains to an antibody that recognizes HLA-DR or a functional fragment thereof. The present invention also relates to a chimeric antigen receptor that has the antibody or a functional fragment thereof, or an immune cell expressing the chimeric antigen receptor. Furthermore, the present invention relates to a multispecific antibody that includes the antibody or a functional fragment thereof and the antigen recognition site for immune cells. Additionally, the present invention relates to a pharmaceutical composition containing these antibodies and the like.

### [Background Art]

Acute myeloid leukemia (AML) is one of the representative blood cancers. If it does not cure with chemotherapy, it becomes an indication for allogeneic hematopoietic stem cell transplantation, and many people are cured by it. However, there are still many who cannot be cured. One promising approach for curing such patients is immunotherapy using chimeric antigen receptor T (CAR-T) cells.

CD19 CAR-T cells have shown significant effects against relapsed/refractory B-cell leukemia and malignant lymphoma (NPLs 1 and 2). However, there is no CAR-T cell that is effective against AML yet. To date, CAR-T cells targeting CD33, CD123, and the like for AML have been developed, but issues such as strong blood toxicity arise because they are expressed in some normal blood cells (NPLs 3 and 4).

Therefore, attempts to search for AML-specific cell surface antigens useful for CAR-T therapy have been made in many places around the world. However, despite intensive efforts using comprehensive methods such as transcriptome analysis or proteome analysis, no good target has been found (NPL 5).

### [Citation List]

### [Non Patent Literature]

[NPL 1] Schuster SJ, Bishop MR, Tam CS, Waller EK, Bo rchmann P, McGuirk JP, et al. Tisagenlecleucel in Adult Relapsed or Refractory Diffuse Large B-Cell Lymphoma. N Engl J Med. 2019; 380(1): 45-56
[NPL 2] Park JH, Riviere I, Gonen M, Wang X, Senechal B, Curran KJ, et al. Long-Term Follow-up of CD19 CAR T herapy in Acute Lymphoblastic Leukemia. N Engl J Med. 2 018; 378(5): 449-59
[NPL 3] Wang, Q.S., Y. Wang, H.Y. Lv, Q.W. Han, H. Fan , B. Guo, L.L. Wang, and W.D. Han.2015.Treatment of CD3 3-directed chimeric antigen receptor-modified T cells i n one patient with relapsed and refractory acute myeloi d leukemia. Mol Ther 23: 184-191
[NPL 4] Loff, S., J. Dietrich, J.E. Meyer, J. Riewald t, J. Spehr, M. von Bonin, C. Grunder, M. Swayampakula, K. Franke, A. Feldmann, M. Bachmann, G. Ehninger, A. E hninger, and M. Cartellieri.2020. Rapidly Switchable Un iversal CAR-T Cells for Treatment of CD123-Positive Leu kemia. Mol Ther Oncolytics 17: 408-420
[NPL 5] Perna F, Berman SH, Soni RK, Mansilla-Soto J, Eyquem J, Hamieh M, et al. Integrating Proteomics and Transcriptomics for Systematic Combinatorial Chimeric Antigen Receptor Therapy of AML. Cancer Cell.2017; 32(4 ): 506-19 e5
[NPL 6] Hasegawa K, Ikeda S, Yaga M, Watanabe K, Urak awa R, Iehara A, et al. Selective targeting of multiple myeloma cells with a monoclonal antibody recognizing t he ubiquitous protein CD98 heavy chain. Sci Transl Med. 2022; 14 (632): eaax7706
[NPL 7] Hosen N, Matsunaga Y, Hasegawa K, Matsuno H, Nakamura Y, Makita M, et al. The activated conformation of integrin beta7 is a novel multiple myeloma-specific target for CAR T cell therapy. Nat Med. 2017; 23(12): 1436-43

### [Summary of Invention]

### [Technical Problem]

The present invention, considering the challenges posed by the aforementioned related art, aims to identify a cell surface antigen specific to blood cancers such as AML and the like, and provide an antibody that recognizes the antigen or a functional fragment thereof as well as means effective for immunotherapy of a blood cancer using the antibody or the like.

### [Solution to Problem]

The present inventors have previously discovered that even if a protein itself is not cancer-specific, the cancer-specific epitope formed by its post-translational change can be a target for immunotherapy (NPLs 6 and 7).

To achieve the aforementioned objective, a multitude of antibodies binding to AML cells were produced, from which AML-specific antibodies were determined to then identify the recognizing antigen thereof. Through this approach, the present inventors aimed for the isolation of AML-specific antibodies, the development of CAR-T cells based thereon, and other tasks.

Specifically, approximately 14,000 hybridomas that secrete monoclonal antibodies (mAb) binding to bone marrow (BM) cells of AML patients or various AML cell lines were established. Next, from these mAbs, 1,078 mAbs that do not bind to cells other than B cells in peripheral blood mononuclear cells (PBMCs) from healthy donors were selected. Further, AML patient BM cells were stained with these candidate mAbs, and 32 clones of mAbs specifically binding to AML were eventually identified.

Then, regarding one of the clones, KG2032, an attempt was made to identify the antigen recognized by the antibody using the loss of binding to lymphoma cells (Daudi cells) with comprehensive gene knockout by the CRISPR-Cas9 system as an indicator. As a result, it was clarified that the antigen recognized by KG2032 was HLA-DR. In addition, 8 clones are among the 32 clones and differ from KG2032, and it was also clarified that an antigen recognized by the 8 clones was HLA-DR.

Furthermore, since HLA-DR is a highly polymorphic molecule, it was investigated which allele of HLA-DR KG2032 could recognize. As a result, it has been revealed that KG2032 binds to chronic myeloid leukemia cell lines (K562 cells) expressing HLA-DRB1*0405, 0410, 0701, 0803, 0901, 1201, or 1454, but KG2032 does not bind to K562 cells expressing HLA-DRB1*0101, 0301, 0403, 0404, 0802, 1101, 1301, 1302, 1403, 1405, 1406, 1501, or 1502.

Also, it has been clarified that KG2032 does not bind to monocytes and some T cells expressing HLA-DR.

Furthermore, the amino acid sequence of the variable region of KG2032 was identified, and a chimeric antigen receptor fused with CD28 and CD3ζ, or CD8α, 4-1BB, and CD3ζ was designed for this region. Then, T cells expressing this chimeric antigen receptor (CAR-T cells) were prepared and cocultured with a cell line derived from human acute myeloid leukemia (KG1a cells). As a result, the CAR-T cells were found to produce cytokines such as IFN-γ and IL-2, and they also exhibited cytotoxic activity. Moreover, when the CAR-T cells were administered to mice transplanted with KG1a cells, a prominent antitumor effect was observed.

Furthermore, a vector for co-expressing the chimeric antigen receptor fused to the variable region of KG2032 with CD28 and CD3ζ and IL-15 was created. Then, NK cells expressing this chimeric antigen receptor (CAR-NK cells) were prepared and cocultured with a cell line derived from human acute myeloid leukemia (KG1a cells). As a result, the CAR-NK cells were found to exhibit cytotoxic activity. Moreover, when the CAR-NK cells were administered to mice transplanted with KG1a cells, a prominent antitumor effect was observed as well.

Based on these results, for the treatment of blood cancer, the present inventors found out that the following use modes (1) and (2) are available by using antibodies such as KG2032 that recognize HLA-DR.
(1) In AML patients with relapse after HLA-DR mismatched allogeneic transplantation, if the HLA-DR of the recipient (leukemia patient) is of the anti HLA-DR antibody -positive type and the HLA-DR type of the donor is of the anti HLA-DR antibody-negative type, KG2032-derived CAR-T cells and the like created from T cells which are derived from the transplant recipient himself/herself or the donor would specifically attack the leukemia of the recipient by using the anti HLA-DR antibody.
(2) By using the anti HLA-DR antibody which only binds to certain cells such as B cells in normal blood cells, and it does not bind to all the hematopoietic stem cells serving as the source of all blood cells, even if conventional autologous CAR-T cells are used for treatment in AML patients with the anti HLA-DR antibody-positive HLA-DR type, leukemia cells would be eliminated, and normal hematopoiesis would be maintained by the anti HLA-DR antibody-negative cells. Thus, it is believed to be viable as a treatment.

This led to the completion of the present invention.

Specifically, the present invention provides the following aspects.
[1] A composition for treating a blood cancer, comprising: an antibody that recognizes HLA-DR or a functional fragment thereof.
[2] A composition to be administered to a blood cancer patient transplanted with hematopoietic stem cells,
   wherein the composition comprises an antibody that recognizes HLA-DR or a functional fragment thereof,
   a donor of the hematopoietic stem cells and the patient have different HLA-DR allele types, and
   the antibody or a functional fragment thereof binds to HLA-DR of the patient but does not bind to HLA-DR of the donor.
[3] The composition according to [1] or [2], wherein the antibody or a functional fragment thereof recognizes HLA-DRB.
[4] The composition according to [1] or [2], wherein the antibody or a functional fragment thereof recognizes HLA-DRB1.
[5] The composition according to [1] or [2], wherein the antibody or a functional fragment thereof binds to at least one allele type of HLA-DR selected from the group consisting of HLA-DRB1*0405, HLA-DRB1*0410, HLA-DRB1*0701, HLA-DRB1*0803, HLA-DRB1*0901, HLA-DRB1*1201, and HLA-DRB1*1454, HLA-DRB1*0101, HLA-DRB1*0301, HLA-DRB1*0403, HLA-DRB1*0404, HLA-DRB1*0802, HLA-DRB1*1101, HLA-DRB1*1301, HLA-DRB1*1302, HLA-DRB1*1403, HLA-DRB1*1405, HLA-DRB1*1406, HLA-DRB1*1501, and HLA-DRB1*1502.
[6] The composition according to [1] or [2], wherein the antibody or a functional fragment thereof binds to an allele type of HLA-DRB1 in which position 86 is an amino acid other than aspartic acid, but does not bind to an allele type of HLA-DRB1 where position 86 is aspartic acid.
[7] The composition according to [1] or [2], wherein the antibody or a functional fragment thereof binds to at least one allele type of HLA-DR selected from the group consisting of HLA-DRB1*0405, HLA-DRB1*0410, HLA-DRB1*0701, HLA-DRB1*0803, HLA-DRB1*0901, HLA-DRB1*1201, and HLA-DRB1*1454, but does not bind to at least one allele type of HLA-DR selected from the group consisting of HLA-DRB1*0101, HLA-DRB1*0301, HLA-DRB1*0403, HLA-DRB1*0404, HLA-DRB1*0802, HLA-DRB1*1101, HLA-DRB1*1301, HLA-DRB1*1302, HLA-DRB1*1403, HLA-DRB1*1405, HLA-DRB1*1406, HLA-DRB1*1501, and HLA-DRB1*1502.
[8] The composition according to any one of [1] to [7] comprising: an immune cell expressing a chimeric antigen receptor that has the antibody or a functional fragment thereof.
[9] The composition according to [8], wherein the immune cell is prepared from a cell derived from the donor of the hematopoietic stem cell.
[10] The composition according to [9], wherein the cell derived from the donor is a pluripotent stem cell.
[11] The composition according to any one of [1] to [7] comprising: a multispecific antibody that has the antibody or a functional fragment thereof and an antigen recognition site for an immune cell.
[12] The composition according to any one of [1] to [7], wherein the antibody or a functional fragment thereof does not bind to some normal cells.
[13] The composition according to any one of [1] to [7], wherein the antibody or a functional fragment thereof does not bind to monocytes.
[14] The composition according to [13] comprising: an immune cell expressing a chimeric antigen receptor that has the antibody or a functional fragment thereof.
[15] The composition according to [13] comprising: a multispecific antibody that has the antibody or a functional fragment thereof and an antigen recognition site for the immune cell.
[16] An antibody that recognizes HLA-DR or a functional fragment thereof, which
   binds to a region containing an amino acid sequence from positions 74 to 89 of HLA-DRB1, where an amino acid at position 86 is an amino acid other than aspartic acid, but
   does not bind to a region containing the amino acid sequence from positions 74 to 89 of HLA-DRB1, where the amino acid at position 86 is aspartic acid.
[17] An antibody that recognizes HLA-DR or a functional fragment thereof, which has a feature described in one of following (a) to (i):
   (a) comprising
      a heavy chain variable region that includes complementarity-determining regions 1 to 3 containing amino acid sequences set forth in SEQ ID NOs: 1 to 3, respectively, and
      a light chain variable region that includes complementarity-determining regions 1 to 3 containing amino acid sequences set forth in SEQ ID NOs: 5 to 7, respectively;
   (b) comprising
      a heavy chain variable region that includes complementarity-determining regions 1 to 3 containing amino acid sequences set forth in SEQ ID NOs: 34 to 36, respectively, and
      a light chain variable region that includes complementarity-determining regions 1 to 3 containing amino acid sequences set forth in SEQ ID NOs: 38 to 40, respectively;
   (c) comprising
      a heavy chain variable region that includes complementarity-determining regions 1 to 3 containing amino acid sequences set forth in SEQ ID NOs: 42 to 44, respectively, and
      a light chain variable region that includes complementarity-determining regions 1 to 3 containing amino acid sequences set forth in SEQ ID NOs: 46 to 48, respectively;
   (d) comprising
      a heavy chain variable region that includes complementarity-determining regions 1 to 3 containing amino acid sequences set forth in SEQ ID NOs: 50 to 52, respectively, and
      a light chain variable region that includes complementarity-determining regions 1 to 3 containing amino acid sequences set forth in SEQ ID NOs: 54 to 56, respectively;
   (e) comprising
      a heavy chain variable region that includes complementarity-determining regions 1 to 3 containing amino acid sequences set forth in SEQ ID NOs: 58 to 60, respectively, and
      a light chain variable region that includes complementarity-determining regions 1 to 3 containing amino acid sequences set forth in SEQ ID NOs: 62 to 64, respectively;
   (f) comprising
      a heavy chain variable region that includes complementarity-determining regions 1 to 3 containing amino acid sequences set forth in SEQ ID NOs: 66 to 68, respectively, and
      a light chain variable region that includes complementarity-determining regions 1 to 3 containing amino acid sequences set forth in SEQ ID NOs: 62 to 64, respectively;
   (g) comprising
      a heavy chain variable region that includes complementarity-determining regions 1 to 3 containing amino acid sequences set forth in SEQ ID NOs: 70 to 72, respectively, and
      a light chain variable region that includes complementarity-determining regions 1 to 3 containing amino acid sequences set forth in SEQ ID NOs: 62 to 64, respectively;
   (h) comprising
      a heavy chain variable region that includes complementarity-determining regions 1 to 3 containing amino acid sequences set forth in SEQ ID NOs: 74 to 76, respectively, and
      a light chain variable region that includes complementarity-determining regions 1 to 3 containing amino acid sequences set forth in SEQ ID NOs: 62 to 64, respectively; and
   (i) comprising
      a heavy chain variable region that includes complementarity-determining regions 1 to 3 containing amino acid sequences set forth in SEQ ID NOs: 66 to 68, respectively, and
      a light chain variable region that includes complementarity-determining regions 1 to 3 containing amino acid sequences set forth in SEQ ID NOs: 62, 78 and 64, respectively.
[18] A chimeric antigen receptor that has the antibody or a functional fragment thereof according to [16] or [17].
[19] An immune cell expressing a chimeric antigen receptor that has the antibody or a functional fragment thereof according to [16] or [17].
[20] The immune cell according to [19], to which the antibody or a functional fragment thereof according to [16] or [17] does not bind.
[21] The immune cell according to [19] or [20], which is at least one immune cell selected from the group consisting of a T cell, an NK cell and an NKT cell.
[22] A pharmaceutical composition comprising: the antibody or a functional fragment thereof according to [16] or [17], or the immune cell according to any one of [19] to [21].
[23] The pharmaceutical composition according to [22], which is a pharmaceutical composition for treating a blood cancer.
[24] The pharmaceutical composition according to [23], wherein the immune cell is prepared from a cell derived from a human who differs from a blood cancer patient subjected to treatment with the pharmaceutical composition.
[25] The pharmaceutical composition according to [23], wherein the immune cell is prepared from a cell collected from a blood cancer patient subjected to treatment with the pharmaceutical composition.
[26] A method for producing the pharmaceutical composition according to [23], comprising the step of modifying an immune cell to which the antibody or a functional fragment thereof according to [16] or [17] does not bind, to express the chimeric antigen receptor according to [18].
[27] The method according to [26], wherein the immune cell is prepared from a cell derived from a human who differs from a blood cancer patient subjected to treatment with the pharmaceutical composition.
[28] The method according to [26], wherein the immune cell is prepared from a cell collected from a blood cancer patient subjected to treatment with the pharmaceutical composition.
[29] The method according to any one of [26] to [28], wherein the immune cell is at least one immune cell selected from the group consisting of a T cell, an NK cell and an NKT cell.

### [Advantageous Effects of Invention]

According to the present invention, by targeting HLA-DR, which is a cell surface antigen specific to a blood cancer, it becomes possible to treat the disease using antibodies that recognize this antigen or functional fragments thereof, or using a chimeric antigen receptor or the like that has it.

In particular, such antibodies can show allele specificity to HLA-DR. For blood cancer patients with relapse after HLA-DR mismatched allogeneic transplantation, if the HLA-DR of the recipient (blood cancer patient) is a positive type that the antibody of the present invention binds to, and the HLA-DR type of the donor is a negative type that the antibody of the present invention does not bind to, then CAR-T cells derived from the antibody of the present invention created from donor-derived cells containing pluripotent stem cells can specifically attack the blood cancer of the recipient.

Furthermore, even if regular autologous CAR-T cells and the like are used for treatment with he antibody of the present invention in blood cancer patients with the positive HLA-DR that the antibody of the present invention binds to, blood cancer cells are eliminated, normal hematopoiesis is maintained by negative cells that the antibody of the present invention does not bind to, making treatment possible.

### [Brief Description of Drawings]

[Fig. 1A] Fig. 1A is a diagram illustrating the results of analyzing the binding of KG2032 to the peripheral blood of healthy individuals by flow cytometry. In the figure, T represents T cells, B represents B cells, Mo represents monocytes, and Neu represents neutrophils.
[Fig. 1B] Fig. 1B is a diagram illustrating the results of analyzing the binding of KG2032 to AML samples by flow cytometry. Unique patient numbers (UPN) 1 to 14 indicate leukemia cells derived from each AML patient.
[Fig. 2A] Fig. 2A is a diagram illustrating an overview of the step to identify the antigen recognized by KG2032 using the CRISPR guide RNA (gRNA) library.
[Fig. 2B] Fig. 2B is a diagram illustrating the results of analyzing the binding of KG2032 to the Cas9-expressing Daudi cells shown in FIG. 2A by flow cytometry. More specifically, after introducing the gRNA library as shown in FIG. 2A, cell fractions with reduced KG2032 binding were sorted. The figure shows the results of analyzing KG2032 binding to cells before and after sorting.
[Fig. 2C] Fig. 2C is a diagram plotting the frequency of the gRNA introduced into the cells before and after the aforementioned sorting.
[Fig. 2D] Fig. 2D is a diagram illustrating the results of analyzing the binding of KG2032 or an existing anti-HLA-DR antibody (L243) to Daudi cells with knocked-out HLA-DR molecule by flow cytometry.
[Fig. 2E] Fig. 2E is a diagram illustrating the results of analyzing the binding of KG2053, KG1982, KG19122, KG19130, KG19214, KG19833, aAML191870 or aAML191872 to Daudi cells with knocked-out HLA-DR molecule by flow cytometry.
[Fig. 3] Fig. 3 is a diagram illustrating the results of analyzing the binding of KG2032 or L243 to K562 cells expressing various HLA-DRB1 molecules by flow cytometry.
[Fig. 4A] Fig. 4A is a diagram illustrating the results of analyzing the binding of KG2032 or L243 to peripheral blood fractions of a healthy individual (Donor 1) with KG2032-negative HLA-DRB1 (0403/0802) or peripheral blood fractions of a healthy individual (Donor 2) with KG2032-positive HLA-DRB1 (0403/0901) by flow cytometry.
[Fig. 4B] Fig. 4B is a diagram illustrating the results of analyzing the binding of KG2032 to bone marrow cells of AML patients with KG2032-positive HLA-DRB1 by flow cytometry.
[Fig. 4C] Fig. 4C is a diagram illustrating the results of analyzing the binding of KG2032 to the fractions of bone marrow cells of a healthy individual with KG2032-positive HLA-DRB1 by flow cytometry.
[Fig. 5A] Fig. 5A is a diagram illustrating an overview of the use mode of KG2032 in AML treatment.
[Fig. 5B] Fig. 5B is a diagram illustrating an overview of another use mode of KG2032 in AML treatment.
[Fig. 6] Fig. 6 is a diagram illustrating the results of comparing the amino acid sequences of DRB1*1454 (KG2032-positive) and DRB1*1502 (KG2032-negative). In the figure, Regions 1 to 5 each indicate an area with many differences in the amino acid sequence.
[Fig. 7A] Fig. 7A is a diagram illustrating an overview of a chimeric protein partially fused with HLA-DRB1*1454 (KG2032-positive) and HLA-DRB1*1502 (KG2032-negative).
[Fig. 7B] Fig. 7B is a diagram illustrating the results of analyzing the binding of KG2032 or L243 to K562 cells expressing the various chimeric proteins by flow cytometry.
[Fig. 7C] Fig. 7C is a diagram illustrating the results of comparing the amino acid sequences of Region 3 for various HLA-DRB1 molecules expressed in K562 cells, between those that KG2032 binds to and those it does not.
[Fig. 7D] Fig. 7D is a diagram illustrating the results of comparing the binding of KG2032 or L243 when expressing a mutant in K562 cells where the amino acid serine (S) at position 86 of DRB1*0405 (KG2032-positive) is substituted by aspartic acid (D).
[Fig. 8A] Fig. 8A is a diagram illustrating an overview of the chimeric antigen receptor (KG2032-CAR) containing the variable region of KG2032.
[Fig. 8B] Fig. 8B is a diagram illustrating the results of analyzing the introduction rate of KG2032-CAR.
[Fig. 8C] Fig. 8C is a graph showing the proliferation rate of T cells expressing KG2032-CAR (KG2032-CAR-T cells). In the figure, "Control" indicates T cells that have not undergone gene introduction.
[Fig. 8D] Fig. 8D is a graph showing the results of analyzing cytokine production after coculturing KG2032-CAR-T cells and leukemia cell lines KG1a (KG2032-positive) or THP-1 (KG2032-negative) for 24 hours.
[Fig. 8E] Fig. 8E is a graph showing the results of analyzing cytotoxic activity after coculturing KG2032-CAR-T cells and leukemia cell lines KG1a or THP-1 for 4 hours.
[Fig. 8F] Fig. 8F is a graph showing the results of analyzing the proliferation of KG2032-CAR-T cells with and without the addition of dasatinib. Note that in the drawings from FIG. 8F onwards, the results using "BBζ" shown in FIG. 8A are presented as KG2032-CAR-T.
[Fig. 8G] Fig. 8G is a diagram illustrating the results of analyzing the expression of T cell exhaustion markers (PD-1, LAG3, and Tim-3) in KG2032-CAR-T cells with and without the addition of dasatinib by flow cytometry.
[Fig. 8H] Fig. 8H is a graph showing the results of analyzing IL2 production in coculture of KG2032-CAR-T cells and KG1a cells with and without the addition of dasatinib.
[Fig. 8I] Fig. 8I is a diagram illustrating the results of analyzing the effects of administering KG2032-CAR-T cells in a xenograft model where luciferase-expressing KG1a cells were transplanted into NOG mice, by detecting bioluminescence using an in vivo imaging system (IVIS).
[Fig. 8J] Fig. 8J is a graph illustrating the results of analyzing the effects of administering KG2032-CAR-T cells in a xenograft model where luciferase-expressing KG1a cells were transplanted into NOG mice. This graph shows the trend of luminescence intensity detected by IVIS.
[Fig. 9A] Fig. 9A is a diagram illustrating overviews of KG2032 CAR-T2A-IL-15 and a process for producing NK cells transduced with the CAR.
[Fig. 9B] Fig. 9B is a graph showing the results of analyzing the proliferation of NK cells transduced with KG2032 CAR-T2A-IL15 (KG2032 CAR-NK) or control (nontransduced (NT)) NK cells during in vitro culture by flow cytometry.
[Fig. 9C] Fig. 9C is a diagram illustrating the results of analyzing the expression of CD56, CD3, and KG2032 CAR in the KG2032 CAR-NK by flow cytometry.
[Fig. 9D] Fig. 9D is a graph showing the results of measuring CD107a degranulation in the KG2032 CAR-NK or control NK cells upon co-culture with the indicated target cells.
[Fig. 9E] Fig. 9E is a diagram illustrating the results of measuring specific lysis of the KG2032 CAR-NK or control NK cells upon co-culture with the indicated target cells by ⁵¹Cr release assay.
[Fig. 9F] Fig. 9F is a diagram illustrating when to transfer KG1a AML cells (KG1a-luc) into immunodeficient mice and infuse the mice with the KG2032 CAR-NK.
[Fig. 9G] Fig. 9G is a graph showing the survival rate of KG1a-transplanted mice to which KG2032 CAR-NK were administered.
[Fig. 9H] Fig. 9H is a diagram illustrating the results of analyzing mice infused with the KG2032 CAR-NK or control NK cells by detecting bioluminescence (n=4 in each group).
[Fig. 10A] Fig. 10A is a diagram illustrating when to transfer BM cells from AML patients into immunodeficient mice and infuse the mice with modified KG2032 CAR-T cells.
[Fig. 10B] Fig. 10B is a diagram illustrating the results of analyzing BM cells of mice infused with the modified KG2032 CAR-T cells or control T cells by flow cytometry. Plots are pre-gated as live mCD45- cells.
[Fig. 10C] Fig. 10C is a dot diagram showing percentages of hCD45+hCD3-hCD34+ AML cells among mCD45-BM cells on 31 days after transplantation of AML cells.

### [Description of Embodiments]

### <Anti-HLA-DR Antibody>

The present invention relates to an antibody that recognizes HLA-DR, which is one of the class II major histocompatibility complexes (MHC), or a functional fragment thereof. In the present invention, "recognizing HLA-DR" means binding to at least one allele type in HLA-DR and also includes binding to at least one allele type in HLA-DR while not binding to at least another allele type.

Examples of the allele type of HLA-DR include HLA-DR1, HLA-DR2, HLA-DR3, HLA-DR4, HLA-DR5, HLA-DR6, HLA-DR7, HLA-DR8, HLA-DR9, HLA-DR10, HLA-DR11, HLA-DR12, HLA-DR13, HLA-DR14, HLA-DR15, HLA-DR52, and HLA-DR53. The HLA-DR may be, for example, a complex of an α-chain and a β-chain. More specifically, examples of the α-chain include HLA-DRA such as HLA-DRA1, and examples of the β-chain include HLA-DRB such as HLA-DRB1, HLA-DRB3, HLA-DRB4, or HLA-DRB5. Moreover, examples of HLA-DRB1 include HLA-DRB1*0405 (SEQ ID NO: 11), HLA-DRB1*0410 (SEQ ID NO: 12), HLA-DRB1*0701 (SEQ ID NO: 13), HLA-DRB1*0803 (SEQ ID NO: 14), HLA-DRB1*0901 (SEQ ID NO: 15), HLA-DRB1*1201 (SEQ ID NO: 16), HLA-DRB1*1454 (SEQ ID NO: 17), HLA-DRB1*0101 (SEQ ID NO: 18), HLA-DRB1*0301 (SEQ ID NO: 19), HLA-DRB1*0403 (SEQ ID NO: 20), HLA-DRB1*0404 (SEQ ID NO: 21), HLA-DRB1*0802 (SEQ ID NO: 22), HLA-DRB1*1101 (SEQ ID NO: 23), HLA-DRB1*1301 (SEQ ID NO: 24), HLA-DRB1*1302 (SEQ ID NO: 25), HLA-DRB1*1403 (SEQ ID NO: 26), HLA-DRB1*1405 (SEQ ID NO: 27), HLA-DRB1*1406 (SEQ ID NO: 28), HLA-DRB1*1501 (SEQ ID NO: 29), and HLA-DRB1*1502 (SEQ ID NO: 30).

For example, an antibody of the present invention is an antibody that binds to an allele type of HLA-DRB1 in which position 86 is an amino acid other than aspartic acid. Moreover, such an antibody may be an antibody that does not bind to an allele type of HLA-DRB1 where position 86 is aspartic acid. Note that the "amino acid other than aspartic acid" at position 86 is preferably serine, valine, or alanine.

More specific examples include antibodies that bind to at least one (for example, 1, 2, 3, 4, 5, 6, or 7) HLA-DR selected from the group consisting of HLA-DRB1*0405 (SEQ ID NO: 11), HLA-DRB1*0410 (SEQ ID NO: 12), HLA-DRB1*0701 (SEQ ID NO: 13), HLA-DRB1*0803 (SEQ ID NO: 14), HLA-DRB1*0901 (SEQ ID NO: 15), HLA-DRB1*1201 (SEQ ID NO: 16), and HLA-DRB1*1454 (SEQ ID NO: 17). On the other hand, examples thereof may also include antibodies that do not bind to at least one (for example, 1, 2, 3, 4, 5, 6, 7, 8, 9, 10, 11, 12, or 13) HLA-DR selected from the group consisting of HLA-DRB1*0101 (SEQ ID NO: 18), HLA-DRB1*0301 (SEQ ID NO: 19), HLA-DRB1*0403 (SEQ ID NO: 20), HLA-DRB1*0404 (SEQ ID NO: 21), HLA-DRB1*0802 (SEQ ID NO: 22), HLA-DRB1*1101 (SEQ ID NO: 23), HLA-DRB1*1301 (SEQ ID NO: 24), HLA-DRB1*1302 (SEQ ID NO: 25), HLA-DRB1*1403 (SEQ ID NO: 26), HLA-DRB1*1405 (SEQ ID NO: 27), HLA-DRB1*1406 (SEQ ID NO: 28), HLA-DRB1*1501 (SEQ ID NO: 29), and HLA-DRB1*1502 (SEQ ID NO: 30). Furthermore, as shown in the Examples described later, the antibody of the present invention can recognize the region containing the amino acid sequence of positions 74 to 89 of HLA-DRB1. Also, the antibody of the present invention may be one that does not bind to monocytes or some T cells.

The presence or absence of binding of the antibody can be analyzed by those skilled in the art using immunological methods. For example, as shown in the Examples described later, in antigen analysis by flow cytometry, if a peak derived from the test antibody shifts to the side with stronger fluorescence intensity compared to a peak derived from its isotype control antibody, it can be determined that the test antibody binds (substantially) to the antigen. Meanwhile, if such a shift is not observed, it can be determined that the test antibody does not bind (substantially) to the antigen.

Furthermore, the binding of the antibody of the present invention is preferably 10⁻⁷ or less in terms of K_{D} (dissociation constant), and more preferably 10⁻⁸ or less. K_{D} is calculated from ka (binding rate constant) and kd (dissociation rate constant) (K_{D} = kd/ka). The terms ka and kd are rate constants in the binding and dissociation reactions between two molecules, and, for example, can be determined by measurements using surface plasmon resonance (SPR). The SPR measurement of the binding between the antibody and the antigen is well-known, and those skilled in the art can determine ka and kd of the antibody based on such well-known technique, and further, can calculate K_{D}.

In the present invention, the term "antibody" encompasses all classes and subclasses of immunoglobulins. "Antibody" includes polyclonal antibodies and monoclonal antibodies. "Polyclonal antibody" is an antibody preparation that contains different antibodies against different epitopes. In contrast, "monoclonal antibody" refers to an antibody obtained from a substantially homogeneous population of antibodies. Unlike polyclonal antibodies, monoclonal antibodies recognize a single determinant on the antigen. The antibody of the present invention is preferably a monoclonal antibody. The antibody of the present invention is an antibody that has been separated and/or recovered (that is, isolated) from components in the natural environment.

The antibody of the present invention may have at least one cytotoxic activity selected from ADCC activity and CDC activity, and may have both ADCC and CDC activities.

In the present invention, "ADCC activity (Antibody-Dependent Cellular Cytotoxicity activity)" refers to the activity where, when an antibody binds to the cell surface antigen of the target cells, effector cells (immune cells such as NK cells and monocytes) further bind to the Fc region of that antibody, activating the cell, which in turn releases factors to kill the target cells. Meanwhile, "CDC activity (Complement-Dependent Cytotoxicity activity)" refers to the activity where, when an antibody binds to the target cells, the complement system is activated, resulting in the lysis of the target cells.

The antibody of the present invention may also have antitumor activity. In the present invention, "antitumor activity" means any one of the activities that inhibit the proliferation of cancer cells, induce the death of cancer cells, and inhibit the metastasis of cancer cells. The antitumor activity can be evaluated, for example, by an analysis using the tumorbearing model as shown in the Examples described later. Furthermore, even without such an in vivo system, if tumor cells are cultured in the presence or absence of the test antibody, and the number of tumor cells in the former is reduced compared to that in the latter, it can be determined that the antibody has antitumor activity.

The antibody of the present invention only needs to recognize HLA-DR, and its origin, type, shape, and the like are not particularly limited. Specific examples thereof include antibodies derived from non-human animals (such as mouse antibodies, rabbit antibodies, rat antibodies, and camel antibodies), human-derived antibodies, chimeric antibodies, and humanized antibodies.

In the present invention, as an example of "antibodies derived from non-human animals," KG2032, KG2053, KG1982, KG19130, KG19214, KG19833, aAML191870, aAML191872, and KG19122 can be mentioned, as shown in the Examples described later. More specifically, antibodies that has a variable region containing the following amino acid sequence can be mentioned:
(a) an antibody that has a heavy chain variable region containing the amino acid sequence set forth in SEQ ID NO: 4, and a light chain variable region containing the amino acid sequence set forth in SEQ ID NO: 8;
(b) an antibody that has a heavy chain variable region containing the amino acid sequence set forth in SEQ ID NO: 37, and a light chain variable region containing the amino acid sequence set forth in SEQ ID NO: 41;
(c) an antibody that has a heavy chain variable region containing the amino acid sequence set forth in SEQ ID NO: 45, and a light chain variable region containing the amino acid sequence set forth in SEQ ID NO: 49;
(d) an antibody that has a heavy chain variable region containing the amino acid sequence set forth in SEQ ID NO: 53, and a light chain variable region containing the amino acid sequence set forth in SEQ ID NO: 57;
(e) an antibody that has a heavy chain variable region containing the amino acid sequence set forth in SEQ ID NO: 61, and a light chain variable region containing the amino acid sequence set forth in SEQ ID NO: 65;
(f) an antibody that has a heavy chain variable region containing the amino acid sequence set forth in SEQ ID NO: 69, and a light chain variable region containing the amino acid sequence set forth in SEQ ID NO: 65;
(g) an antibody that has a heavy chain variable region containing the amino acid sequence set forth in SEQ ID NO: 73, and a light chain variable region containing the amino acid sequence set forth in SEQ ID NO: 65;
(h) an antibody that has a heavy chain variable region containing the amino acid sequence set forth in SEQ ID NO: 77, and a light chain variable region containing the amino acid sequence set forth in SEQ ID NO: 65;
(i) an antibody that has a heavy chain variable region containing the amino acid sequence set forth in SEQ ID NO: 69, and a light chain variable region containing the amino acid sequence set forth in SEQ ID NO: 79.

Furthermore, antibodies that recognize HLA-DR and have the following complementarity-determining regions (CDRs) can also be given as an example:
(a) an antibody that has heavy chain CDRs 1 to 3 determined from the heavy chain variable region containing the amino acid sequence set forth in SEQ ID NO: 4, and light chain CDRs 1 to 3 determined from the light chain variable region containing the amino acid sequence set forth in SEQ ID NO: 8;
(b) an antibody that has heavy chain CDRs 1 to 3 determined from the heavy chain variable region containing the amino acid sequence set forth in SEQ ID NO: 37, and light chain CDRs 1 to 3 determined from the light chain variable region containing the amino acid sequence set forth in SEQ ID NO: 41;
(c) an antibody that has heavy chain CDRs 1 to 3 determined from the heavy chain variable region containing the amino acid sequence set forth in SEQ ID NO: 45, and light chain CDRs 1 to 3 determined from the light chain variable region containing the amino acid sequence set forth in SEQ ID NO: 49;
(d) an antibody that has heavy chain CDRs 1 to 3 determined from the heavy chain variable region containing the amino acid sequence set forth in SEQ ID NO: 53, and light chain CDRs 1 to 3 determined from the light chain variable region containing the amino acid sequence set forth in SEQ ID NO: 57;
(e)an antibody that has heavy chain CDRs 1 to 3 determined from the heavy chain variable region containing the amino acid sequence set forth in SEQ ID NO: 61, and light chain CDRs 1 to 3 determined from the light chain variable region containing the amino acid sequence set forth in SEQ ID NO: 65;
(f) an antibody that has heavy chain CDRs 1 to 3 determined from the heavy chain variable region containing the amino acid sequence set forth in SEQ ID NO: 69, and light chain CDRs 1 to 3 determined from the light chain variable region containing the amino acid sequence set forth in SEQ ID NO: 65;
(g)an antibody that has heavy chain CDRs 1 to 3 determined from the heavy chain variable region containing the amino acid sequence set forth in SEQ ID NO: 73, and light chain CDRs 1 to 3 determined from the light chain variable region containing the amino acid sequence set forth in SEQ ID NO: 65;
(h)an antibody that has heavy chain CDRs 1 to 3 determined from the heavy chain variable region containing the amino acid sequence set forth in SEQ ID NO: 77, and light chain CDRs 1 to 3 determined from the light chain variable region containing the amino acid sequence set forth in SEQ ID NO: 65;
(i) an antibody that has heavy chain CDRs 1 to 3 determined from the heavy chain variable region containing the amino acid sequence set forth in SEQ ID NO: 69, and light chain CDRs 1 to 3 determined from the light chain variable region containing the amino acid sequence set forth in SEQ ID NO: 79.

Note that regarding such antibodies, there is no particular limitation on the method for determining the CDR based on the amino acid sequence of the variable region. However, known numbering schemes such as Kabat, Chothia, IMGT, and Aho can be mentioned. More specifically, an example determined by the Kabat, antibodies that recognize HLA-DR and has the following CDR can also be given as examples:
(a) an antibody that has the amino acid sequences set forth in SEQ ID NOs: 1 to 3 as heavy chain CDRs 1 to 3, respectively, and the amino acid sequences set forth in SEQ ID NOs: 5 to 7 as light chain CDRs 1 to 3, respectively;
(b) an antibody that has the amino acid sequences set forth in SEQ ID NOs: 34 to 36 as heavy chain CDRs 1 to 3, respectively, and the amino acid sequences set forth in SEQ ID NOs: 38 to 40 as light chain CDRs 1 to 3, respectively;
(c) an antibody that has the amino acid sequences set forth in SEQ ID NOs: 42 to 44 as heavy chain CDRs 1 to 3, respectively, and the amino acid sequences set forth in SEQ ID NOs: 46 to 48 as light chain CDRs 1 to 3, respectively;
(d) an antibody that has the amino acid sequences set forth in SEQ ID NOs: 50 to 52 as heavy chain CDRs 1 to 3, respectively, and the amino acid sequences set forth in SEQ ID NOs: 54 to 56 as light chain CDRs 1 to 3, respectively;
(e) an antibody that has the amino acid sequences set forth in SEQ ID NOs: 58 to 60 as heavy chain CDRs 1 to 3, respectively, and the amino acid sequences set forth in SEQ ID NOs: 62 to 64 as light chain CDRs 1 to 3, respectively;
(f) an antibody that has the amino acid sequences set forth in SEQ ID NOs: 66 to 68 as heavy chain CDRs 1 to 3, respectively, and the amino acid sequences set forth in SEQ ID NOs: 62 to 64 as light chain CDRs 1 to 3, respectively;
(g) an antibody that has the amino acid sequences set forth in SEQ ID NOs: 70 to 72 as heavy chain CDRs 1 to 3, respectively, and the amino acid sequences set forth in SEQ ID NOs: 62 to 64 as light chain CDRs 1 to 3, respectively;
(h) an antibody that has the amino acid sequences set forth in SEQ ID NOs: 74 to 76 as heavy chain CDRs 1 to 3, respectively, and the amino acid sequences set forth in SEQ ID NOs: 62 to 64 as light chain CDRs 1 to 3, respectively;
(i) an antibody that has the amino acid sequences set forth in SEQ ID NOs: 66 to 68 as heavy chain CDRs 1 to 3, respectively, and the amino acid sequences set forth in SEQ ID NOs: 62, 78 and 64 as light chain CDRs 1 to 3, respectively.

The present invention also includes not only antibodies having such specific amino acid sequences forming variable regions and/or CDRs, but also antibodies whose amino acid sequence is modified without reducing the desired activity (such as reactivity against antigens). Such amino acid sequence variants can be prepared, for example, by introducing mutations into the DNA encoding the aforementioned variable regions, or by peptide synthesis. Such modifications include, for example, substitution, deletion, addition, and/or insertion of residues within the amino acid sequence of the antibody. The site of modification in the amino acid sequence of the antibody may be in the constant region of the heavy or light chain of the antibody, as long as it has equivalent activity to the antibody before modification, or may also be in the variable region (framework region (FR) and CDR). Modifications to amino acids other than the CDR are typically made to the FR because they are believed to have relatively little impact on reactivity with the antigen. However, it is known at present that methods for modifying the amino acids of CDRs and screening for antibodies with enhanced affinity for antigens are available (PNAS, 102: 8466-8471 (2005), Protein Engineering, Design & Selection, 21: 485-493 (2008), International Publication No. WO2002/051870, J. Biol. Chem., 280: 24880-24887 (2005), Protein Engineering, Design & Selection, 21: 345-351 (2008), MAbs, Mar-Apr; 6(2): 437-45 (2014)). Also, by using an integrated computational chemistry system (such as Molecular Operating Environment manufactured by CCG in Canada), it is now possible to model antibodies with enhanced affinity to antigens (see http://www.rsi.co.jp/kagaku/cs/ccg/products/applicatio n/protein.html). Furthermore, as described in Protein Eng Des Sel. 2010 Aug; 23(8): 643-51, there is a known example where in terms of affinity to antigens, the CDR1 of the heavy chain variable region and the CDR3 of the light chain variable region are not involved. Similarly, it is reported in Molecular Immunology 44: 1075-1084 (2007) that in most antibodies, the CDR2 of the light chain variable region is not involved in affinity for the antigen. In this way, in terms of an antibody's affinity for antigens, not all of the CDR1 to 3 of both the heavy chain variable region and the light chain variable region are required in order to exhibit equivalent activity. In fact, examples where the affinity for antigens was maintained by having at least one CDR of the original antibody have been reported in Biochem Biophys Res Commun. 2003 Jul 18; 307(1): 198-205, J Mol Biol. 2004 Jul 9; 340(3): 525-42, and J Mol Biol. 2003 Aug 29; 331(5): 1109-20.

Regarding the antibody of the present invention, the number of amino acids modified in the variable region is preferably within 10 amino acids, more preferably within 5 amino acids, and further preferably within 3 amino acids (for example, within 2 amino acids, or 1 amino acid). Furthermore, all such modifications are preferably made in regions other than CDR, namely in the FR, from the viewpoint of minimizing the impact on binding to the antigen. Also, the number of amino acids modified in the CDR is preferably within 5 amino acids, and more preferably within 3 amino acids (for example, within 2 amino acids, or 1 amino acid).

The modification of amino acids is preferably a conservative substitution. In the present invention, "conservative substitution" refers to the substitution with another similar (having a chemically equivalent side chain) amino acid residue. Groups of amino acid residues having chemically similar side chains are well known in the art to which the present invention belongs. For example, they can be categorized as acidic amino acids (aspartic acid and glutamic acid), basic amino acids (lysine, arginine, and histidine), and neutral amino acids, namely amino acids with hydrocarbon chains (glycine, alanine, valine, leucine, isoleucine, and proline), amino acids with hydroxyl groups (serine and threonine), sulfur-containing amino acids (cysteine and methionine), amino acids with amide groups (asparagine and glutamine), amino acids with an imino group (proline), and amino acids with aromatic groups (phenylalanine, tyrosine, and tryptophan).

Additionally, regarding the amino acid sequence after modification, the antibodies of the present invention also include an antibody that has a variable region containing an amino acid sequence having 80% or more homology or identity at the amino acid sequence level with the aforementioned specific amino acid sequence, as long as it has equivalent activity to the antibody before the modification. Such homology or identity may be at least 80%, but preferably 85% or more, more preferably 90% or more, and further preferably 95% or more (for example, 96% or more, 97% or more, 98% or more, or 99% or more). Note that modifications in the variable region of such antibodies are preferably made in regions other than CDR, namely in the FR, from the viewpoint of minimizing the impact on binding to the antigen. Also, "identity" means the proportion of identical amino acid types between the compared amino acid sequences, and "homology" means the sum of the proportion of similar amino acids and the proportion of identical ones. The sequence homology or identity can be determined using the BLASTP (amino acid level) program (Altschul et al., J. Mol. Biol., 215: 403-410, 1990). This program is based on the BLAST algorithm by Karlin and Altschul (Proc. Natl. Acad. Sci. USA, 87: 2264-2268, 1990; and Proc. Natl. Acad. Sci. USA, 90: 5873-5877, 1993). When analyzing amino acid sequences with BLASTP, parameters can be set, for instance, score = 50 and word length = 3. Also, amino acid sequences can be analyzed using the Gapped BLAST program as described by Altschul et al. (Nucleic Acids Res. 25: 3389-3402, 1997). When using BLAST and Gapped BLAST programs, the default parameters of each program can be used. Specific procedures for these analysis methods are well known.

Moreover, "having equivalent activity" means, for example, that the binding to the antigen is equivalent (for example, 70% or more, preferably 80% or more, or more preferably 90% or more) to the target antibody (for example, KG2032, KG2053, KG1982, KG19130, KG19214, KG19833, aAML191870, aAML191872, or KG19122).

The modification of the antibody of the present invention may include, for example, modifications to the post-translational processes of the antibody, such as changing the number or position of glycosylation sites. This can, for example, enhance the ADCC activity of the antibody. Glycosylation of an antibody typically involves N-linkage or O-linkage. The glycosylation of an antibody largely depends on the host cell used for expressing the antibody. Modifications to the glycosylation pattern can be made by known methods, such as the introduction or loss of specific enzymes related to sugar production (Japanese Patent Application Publication No. 2008-113663, US Patent No. 5047335, US Patent No. 5510261, US Patent No. 5278299, and International Publication No. WO99/54342). Furthermore, in the present invention, deamidation of an amino acid or an amino acid adjacent to a deamidated amino acid may be inhibited by substitution with another amino acid to increase the stability of the antibody or for other purposes. It is also possible to substitute glutamic acid with another amino acid to increase the stability of the antibody. The present invention even provides such stabilized antibodies.

In the present invention, a "chimeric antibody" refers to an antibody formed by linking the variable region of a certain antibody with the constant region of a different antibody. For example, a chimeric antibody can be obtained by immunizing a mouse with an antigen, extracting the variable part (variable region) of the antibody that binds to the antigen from the genes of the mouse monoclonal antibody, binding it to the constant part (constant region) gene of the antibody derived from human bone marrow, incorporating this into an expression vector, and introducing it into a host for production (Japanese Patent Application Publication No. Hei 8-280387, US Patent No. 4816397, US Patent No. 4816567, and US Patent No. 5807715).

Typically, the constant region of a chimeric antibody used is that of a human-derived antibody. For example, in the heavy chain, Cγ1, Cγ2, Cγ3, Cγ4, Cµ, Cδ, Cα1, Cα2, and Cε can be used as the constant regions. In the light chain, Cκ and Cλ can be used as the constant regions. The amino acid sequences of these constant regions, as well as the base sequences encoding them, are known. Moreover, to improve the stability of the antibody itself or the stable production of the antibody, one or several amino acids in the human-derived antibody constant region can be substituted, deleted, added, and/or inserted.

In the present invention, a "humanized antibody" refers to an antibody where the genetic sequence of the CDR of an antibody derived from a non-human animal has been transplanted to a human-derived antibody gene (CDR grafting), and the production method thereof is known, such as overlap extension PCR (for example, European Patent Application Publication No. 239400, European Patent Application Publication No. 125023, International Publication No. WO90/07861, and International Publication No. WO96/02576). The variable region of an antibody is usually composed of three CDRs flanked by four FRs. The CDR is a region that substantially determines the binding specificity of the antibody. While the amino acid sequence of the CDR is diverse, the amino acid sequence forming the FR often shows high homology or identity even among antibodies with different binding specificities. Therefore, it is generally said that by transplanting the CDR, the binding specificity of one antibody can be transferred to another antibody. From the viewpoint of maintaining the functions of the CDR, when transplanting the non-human derived CDR to human FR, a human FR with high homology or identity to the FR derived from the non-human animal is selected. That is, amino acids within the CDR not only recognize the antigen but also coordinate with the amino acids in the FR near the CDR and are also involved in maintaining the loop structure of the CDR, so that it is preferable to use a human FR composed of an amino acid sequence with high homology or identity to the amino acid sequence of the FR adjacent to the CDR to be transplanted.

The search for a known human FR with high homology or identity to the FR derived from a non-human animal can be conducted, for example, using an antibodyspecific search system available on the internet (http://www.bioinf.org.uk/abysis/). To match the sequence of the thus obtained human FR, mutations can be introduced into sequences other than the CDR of the non-human derived antibody. Alternatively, if the gene (cDNA) encoding the amino acid sequence of the human FR obtained by the search is available, the non-human derived CDR may be introduced into that sequence. Mutations can be introduced using known techniques in the art, such as nucleic acid synthesis and sitespecific mutagenesis.

The binding activity of the thus produced humanized antibody to antigens can be qualitatively or quantitatively measured and evaluated, thereby making it possible to appropriately select a human-derived antibody FR where, when linked via a CDR, the CDR forms a good antigen-binding site. If necessary, the amino acid residues of the FR of the humanized antibody can be substituted so that the CDR forms an appropriate antigen-binding site as described in methods such as those in Sato, K. et al., Cancer Res, 1993, 53, 851-856, and furthermore, by measuring and evaluating the binding activity of the mutated antibody with the substituted amino acid to antigens, a mutated FR sequence with the desired properties can be selected.

Further, the antibody of the present invention may be in the form of an antibody that competes in the binding to HLA-DR with an antibody that has a variable region or CDR containing the aforementioned specific amino acid sequence or a variant thereof. In other words, an antibody may be in the form of an antibody that binds to an epitope showing binding properties with an antibody having a variable region or CDR containing the aforementioned specific amino acid sequence or a variant thereof.

In the present invention, the term "epitope" refers to the antigenic determinant present in the antigen, that is, the site on the antigen that the antigen-binding domain in the antibody binds to. Therefore, the epitope in the present invention may be a polypeptide composed of multiple amino acids that are continuous in the primary amino acid sequence (linear epitope) or a polypeptide formed by amino acids that are not adjacent in the primary amino acid sequence coming close due to the three-dimensional structure such as peptide or protein folding (discontinuous epitope or structural epitope). Such epitopes are typically composed of at least 3 (for example, 4) amino acids, and most commonly composed of at least 5 (for example, 6-20, 7-15, 8-10) amino acids.

In addition, if an antibody is obtained, those skilled in the art can identify the peptide region (epitope) on the antigen where the antibody shows reactivity and can prepare various antibodies that bind to that peptide region. Furthermore, whether two antibodies bind to the same or sterically overlapping epitopes can be determined by competitive assay.

In the present invention, the "functional fragment" of an antibody refers to a part (partial fragment) of the antibody that recognizes HLA-DR. Specifically, this includes Fab, Fab', F(ab')2, variable fragment (Fv), disulfide-bonded Fv, single-chain variable fragment (single-chain Fv, scFv), sc(Fv)2, and polymers thereof, among others.

Here, "Fab" refers to a monovalent antigen-binding fragment of an immunoglobulin composed of one light chain and a part of a heavy chain. It can be obtained by papain digestion of the antibody or by recombinant methods. "Fab‴ is different from Fab in that it includes one or more cysteines in the hinge region of the antibody and a slight number of residues added at the carboxy terminus of the CH1 domain of the heavy chain. "F(ab')2" refers to a divalent antigen-binding fragment of an immunoglobulin composed of parts of both light chains and both heavy chains.

The "variable fragment (Fv)" is the smallest antibody fragment that possesses a complete antigen recognition and binding site. Fv is a dimer in which the heavy chain variable region and light chain variable region are strongly linked by non-covalent bonds. The "single-chain variable fragment (single-chain Fv, scFv)" contains the heavy chain variable region and light chain variable region of the antibody, and these regions exist on a single polypeptide chain. "sc (Fv) 2" is composed of two heavy chain variable regions and two light chain variable regions linked together into a single chain by linkers or the like.

The antibody of the present invention can be produced by the hybridoma method and can also be produced by the recombinant DNA method. As for the hybridoma method, a representative method is that of Köhler and Milstein (Köhler & Milstein, Nature, 256: 495 (1975)). The antibody-producing cells used in the cell fusion step in this method are spleen cells, lymph node cells, peripheral blood leukocytes, and other cells of animals (such as mice, rats, hamsters, rabbits, monkeys, and goats) immunized with an antigen (such as HLA-DR, partial peptides thereof, proteins obtained by fusing them with Fc protein or the like, or cells expressing these). It is also possible to use antibody-producing cells obtained by acting an antigen on the above cells or lymphocytes previously isolated from unimmunized animals in a culture medium. Various known cell lines can be used as myeloma cells. Antibody-producing cells and myeloma cells may originate from different animal species as long as they can fuse, but preferably, they originate from the same animal species. Hybridomas can be produced by cell fusion between spleen cells obtained from a mouse immunized with the antigen and mouse myeloma cells, and thereafter, by screening, a hybridoma that produces monoclonal antibodies recognizing HLA-DR can be obtained. Monoclonal antibodies recognizing HLA-DR can be obtained by culturing the hybridomas or from the ascites of a mammal to which the hybridoma has been administered.

The recombinant DNA method is a technique to clone DNA encoding the antibody of the present invention from hybridomas, B cells, or the like, incorporate it into an appropriate vector, introduce it into a host cell (such as mammalian cell lines such as HEK cells, E. coli, yeast cells, insect cells, and plant cells), and produce the antibody of the present invention as a recombinant antibody (for example, P. J. Delves, Antibody Production: Essential Techniques, 1997; WILEY, P. Shepherd and C. Dean Monoclonal Antibodies, 2000, OXFORD UNIVERSITY PRESS; and Vandamme A. M. et al., Eur. J. Biochem. 192: 767-775 (1990)). In the expression of DNA encoding the antibody of the present invention, DNA encoding the heavy chain or light chain may be separately incorporated into expression vectors to transform host cells, or DNA encoding both the heavy chain and light chain may be incorporated into a single expression vector to transform host cells (see International Publication No. WO94/11523). The antibody of the present invention can be obtained in a substantially pure and uniform form by culturing the host cells and separating and purifying it from within the host cells or from the culture fluid. The separation and purification of the antibody can be done using methods commonly used for polypeptide purification. By using transgenic animal preparation techniques, if a transgenic animal (such as cows, goats, sheep, or pigs) containing the antibody gene is prepared, monoclonal antibodies derived from the antibody gene can also be obtained in large quantities from the milk of that transgenic animal.

The present invention can also provide DNA encoding the antibody of the present invention, a vector containing the DNA, a host cell having the DNA, and a method for producing antibodies that includes culturing the host cell and collecting the antibodies.

### <Chimeric Antigen Receptor>

As shown in the Examples described later, the target of the antibody of the present invention, HLA-DR, becomes a cell surface antigen specific to a blood cancer. Also, the variable region of the antibody of the-present invention is useful in chimeric antigen receptor (CAR) and T cells expressing it (so-called CAR-T). Therefore, the present invention can also take the form of a CAR that includes an antibody or that binds to HLA-DR the variable region thereof, a transmembrane domain, and an intracellular signaling domain.

In the present invention, the "chimeric antigen receptor (CAR)" means a chimeric protein in which a region binding to the extracellular region HLA-DR, a transmembrane domain, and an intracellular signaling domain are arranged in that order from the N-terminal side, directly or indirectly connected.

In the present invention, the "region binding to HLA-DR (antigen-binding domain)" can, for example, use the aforementioned antibody or a functional fragment thereof, but scFv is usually used in CAR.

"scFv" is, as mentioned above, a structure in which the light chain variable region and heavy chain variable region of a monoclonal antibody (immunoglobulin) are linked through a linker, and retains the ability to bind to an antigen. In scFv, the order from the N-terminal side can be the light chain variable region, linker, and heavy chain variable region, or it can be the heavy chain variable region, linker, and light chain variable region.

As the "linker" in scFv, for example, a peptide linker can be used. The length of the linker is not particularly limited. For example, a linker with a number of amino acids ranging from 5 to 25 can be used. The length of the linker is preferably 8 to 25 amino acids, and further preferably 15 to 20 amino acids. Suitable examples of peptide linkers include peptide linkers containing glycine or glycine and serine (such as GGS linker, GS linker, GGG linker, and Whitlow/218 linker). The amino acids that constitute these linkers, glycine and serine, have the advantage of being small in size themselves and difficult to form a higher-order structure within the linker.

For the monoclonal antibody that forms the basis of scFv, there are no particular limitations, but examples thereof include the aforementioned non-human animal-derived antibodies, human antibodies, and humanized antibodies. Suitable examples thereof are also as mentioned above.

In the present invention, the term "transmembrane domain" is not particularly limited as long as it refers to a polypeptide that has the function to penetrate the cell membrane and can anchor CAR to the cell membrane. Proteins from which the polypeptide bearing the transmembrane domain is derived include, for example, CD28, CD3ζ, CD3ε, 4-1BB, CD45, CD4, CD5, CD8α, CD8β, CD9, -CD16, CD22, CD33, CD37, CD64, CD80, CD86, CD134, CD137, ICOS, CD154, GITR, and the α and β chains of the T-cell receptor.

In the present invention, the term "intracellular signaling domain" refers to the area located inside the cell when the CAR is positioned on the cell membrane, and this area is capable of transmitting signals necessary for the effector function of immune cells, that is, when the antigen-binding domain binds to the antigen (HLA-DR), it can transmit the signal (the so-called primary signal) necessary for the activation of immune cells. Examples of such intracellular signaling domains typically include those with only the activation signal transmission domain, such as the T-cell receptor (TCR) and CD3 complex (first generation CAR), those that, in addition to the activation signal transmission domain, have a costimulatory signal transmission domain derived from costimulatory molecules (such as the intracellular domain of CD28 or 4-1BB) (second generation CAR), and those that, in addition to the activation signal transmission domain, have multiple costimulatory signal transmission domains (such as the intracellular domains of CD28 and 4-1BB) (third generation CAR). There are also those that include the intracellular regions of CD79A and CD40 (see Mol Ther 2021 Sep 1; 29(9): 2677-2690).

The "intracellular signaling domain," as mentioned above, only needs to be able to transmit the primary signal, and can use the activation signal transmission domain of proteins involved in such signaling. It is known that the immunoreceptor tyrosine-based activation motif (ITAM) is involved in the primary signal transmission. Proteins with ITAM include, for example, CD3ζ, FcRγ, FcRβ, CD3γ, CD3δ, CD3ε, CD5, CD22, CD79a, CD79b, CD66d, DAP10, and DAP12.

In the present invention, the number of intracellular signaling domains included in the CAR is not limited to 1 and can include multiple intracellular signaling domains (for example, 1 to 5, 1 to 3, or 1 or 2 signal transmission factors). In this case, the multiple included intracellular signaling domains may be the same or different.

The CAR of the present invention, in addition to the aforementioned antigen-binding domain, transmembrane domain, and intracellular signaling domain, can also include other domains. Examples of other domains include, but are not limited to, costimulatory transmission domains, spacer sequences, and signal peptides.

The costimulatory molecules expressed on T cells are known to assist in activating T cells by transmitting a costimulatory signal inside the cell through binding to ligands, specific to each costimulatory molecule, expressed on antigen-presenting cells (secondary signal transmission). In the present invention, the term "costimulatory transmission domain" refers to the intracellular domain involved in the costimulatory signal transmission of such costimulatory molecules. Examples of costimulatory molecules include CD28, 4-1BB (CD137), OX40 (CD134), ICOS, CD2, CD4, CD5, CD8α, CD8β, CD154, etc. The CAR of the present invention can include the costimulatory transmission domain of these costimulatory molecules.

The number of costimulatory transmission domains that the CAR of the present invention may contain is not limited to one and can be multiple. The CAR of the present invention can include, for example, 1 to 5, 1 to 3, or 1 or 2 costimulatory transmission domains. When the CAR of the present invention contains multiple costimulatory signal transmission domains, such domains can be the same or different.

In the present invention, the term "spacer" refers to a sequence that connects between each region, etc. There are no particular limitations as long as it does not inhibit the function of each region, and there is no specific limit on the number of amino acids. However, it is typically between 1 and 500 amino acids, preferably 5 to 300 amino acids, and further preferably 10 to 100 amino acids.

While the sequence of the spacer is not particularly limited, the spacer used can be, for example, the hinge portion of IgG, preferably human IgG (such as subtype IgG1 or IgG4), or a part thereof, the hinge portion and a part of CH2, or a part of the factor (for instance, CD28) used for the transmembrane domain. It is anticipated that using the sequence of the hinge portion or a part thereof will result in a flexible spacer.

In the present invention, the term "signal peptide" refers to a peptide for promoting the secretion of CAR or for directing its localization to the cell membrane and is also referred to as a leader sequence. The signal peptide can typically be directly or indirectly attached to the N-terminus of the antigen-binding domain. Optionally, the signal peptide may be cleaved from the antigen-binding domain during cell processing and CAR localization to the cell membrane. Examples of such signal peptides include signal peptides from immunoglobulin (such as IgK), human GM-CSF receptor, T-cell receptor alpha and beta chains, CD8α, CD8β, CD3ζ, CD28, CD3ε, CD45, CD4, CD5, CD9, CD16, CD22, CD33, CD37, CD64, CD80, CD86, CD134, CD137, ICOS, CD154, GITR, etc.

Above, examples of each region and the like in the CAR of the present invention have been described. Specific amino acid sequences related to these regions and the like can be appropriately obtained by those skilled in the art by searching known literature or databases such as NCBI (http://www.ncbi.nlm.nih.gov/guide/). Furthermore, specific sequences are also disclosed in the sequence listing of the present application. Besides these typical amino acid sequences (for example, NCBI reference sequences), regions and the like according to the present invention can include variants and homologs of those typical amino acid sequences as long as they maintain the functions of the respective regions. Such variants and homologs typically have high homology or identity to the typical amino acid sequences. High homology or identity is usually 60% or more, preferably 70% or more, more preferably 80% or more, and further preferably 90% or more (for example, 95% or more, 96% or more, 97% or more, 98% or more, and 99% or more).

Furthermore, the CAR of the present invention may be a molecule composed of one type of polypeptide or may be a molecule composed of a complex of two or more polypeptides. Also, the CAR of the present invention may be a molecule composed of a polypeptide or a complex thereof, or may be one formed by connecting other substances (such as example, fluorescent substances, radioactive substances, and inorganic particles) to a polypeptide or a complex thereof.

The CAR of the present invention may be chemically modified. The polypeptide constituting the CAR of the present invention may have a C-terminus that is a carboxyl group (-COOH), carboxylate (-COO-), amide (-CONH₂), or ester (-COOR). Here, examples of R in the ester used include C1-6 alkyl groups such as methyl, ethyl, n-propyl, isopropyl, and n-butyl; C3-8 cycloalkyl groups such as cyclopentyl and cyclohexyl; C6-12 aryl groups such as phenyl and α-naphthyl; phenyl-C1-2 alkyl groups such as benzyl and phenethyl; α-naphthyl-C1-2 alkyl groups such as α-naphthylmethyl and other C7-14 aralkyl groups; and pivaloyloxymethyl groups. The polypeptide constituting the CAR of the present invention may have its carboxyl group (or carboxylate) other than the C-terminus amidated or esterified. In this case, esters such as those mentioned for the C-terminus are used. Furthermore, the polypeptide constituting the CAR of the present invention also includes those in which the amino group of the N-terminal amino acid residue is protected by a protecting group (for example, formyl group, acetyl group, and other C1-6 acyl groups like C1-6 alkanoyl groups), those in which the N-terminal glutamine residue, which can be cleaved and produced in vivo, has undergone pyroglutamic oxidation, and those in which substituents on the side chain of amino acids within the molecule (such as -OH, -SH, amino groups, imidazole groups, indole groups, or guanidino groups) are appropriately protected by protecting groups (for example, formyl group, acetyl group, and other C1-6 acyl groups like C1-6 alkanoyl groups).

Furthermore, the CAR of the present invention may have additional functional proteins. There are no particular limitations on the additional functional proteins, and they are selected appropriately according to the function desired to be imparted to the CAR of the present invention. For example, functional proteins used to facilitate the purification and detection of CAR include Myc tag, His tag, HA tag, FLAG tag (registered trademark, Sigma-Aldrich), fluorescent protein tags (such as GFP).

The chimeric antigen receptor of the present invention may also be in the form of a pharmaceutically acceptable salt with an acid or base. As long as the salt is a pharmaceutically acceptable salt, it is not particularly limited and can employ either acidic or basic salts. Examples of acidic salts include inorganic acid salts such as hydrochloride, hydrobromide, sulfate, nitrate, and phosphate; organic acid salts such as acetate, propionate, tartrate, fumarate, maleate, malate, citrate, methanesulfonate, p-toluenesulfonate; and amino acid salts such as aspartate and glutamate. Additionally, examples of basic salts include alkali metal salts such as sodium salts and potassium salts; alkaline earth metal salts such as calcium salts and magnesium salts. The chimeric antigen receptor of the present invention may also be in the form of a solvate. Solvents are not particularly limited as long as they are pharmaceutically acceptable, and examples thereof include water, ethanol, glycerol, and acetic acid.

Moreover, there have been a number of reports of experiments and clinical studies using CAR (such as Rossig C, et al., Mol Ther 10: 5-18, 2004; Dotti G, et al., Hum Gene Ther 20: 1229-1239, 2009; Ngo MC, et al., Hum Mol Genet 20 (R1) : R93-99, 2011; Ahmed N, et al., Mol Ther 17: 1779-1787, 2009; Pule MA, et al., Nat Med 14: 1264-1270, 2008; Louis CU, et al., Blood 118: 6050-6056, 2011; Kochenderfer JN, et al., Blood 116: 4099-4102, 2010; Kochenderfer JN, et al., Blood 119: 2709-2720, 2012; Porter DL, et al., N Engl J Med365: 725-733, 2011; Kalos M, et al., Sci Transl Med 3: 95ra73, 2011; Brentjens RJ, et al., Blood 118: 4817-4828, 2011; Brentjens RJ, et al., Sci Transl Med 5: 177 ra38, 2013), and these reports can be used as a basis to construct the CAR of the present invention. Also, since the specific sequences are provided in the Examples described later, the CAR of the present invention can be constructed by referring to them.

### <Nucleotide Encoding Chimeric Antigen Receptor>

The present invention provides a polynucleotide encoding the CAR of the present invention. Information on nucleotide sequences encoding antigen-binding domains, such as scFv, can be obtained, for example, by analyzing nucleotide sequences of hybridomas that produce the monoclonal antibodies they are based on.

Additionally, the specific nucleotide sequences encoding regions other than the antigen-binding domain in the CAR of the present invention can be appropriately obtained by those skilled in the art by searching known literature or databases such as NCBI (http://www.ncbi.nlm.nih.gov/guide/). Moreover, the nucleotide sequences encoding the aforementioned regions are not limited to those known, and any nucleotide sequences encoding those regions and the like can be used. Due to the degeneracy of the genetic code, multiple codons exist for one amino acid. Therefore, numerous nucleotide sequences exist that encode the same amino acid sequence. The nucleotide sequence of the polynucleotide of the present invention may be any of the multiple nucleotide sequences resulting from the degeneracy of the genetic code as long as it encodes the CAR of the present invention. Additionally, to optimize expression in cells that express CAR, the codons selected for coding amino acids in the nucleotide sequences encoding each region in the CAR of the present invention may be modified.

Moreover, those skilled in the art can produce polynucleotides encoding each region using known techniques based on such nucleotide sequence information, such as chemical synthesis methods (such as the phosphoramidite method) or PCR amplification methods. Furthermore, the polynucleotides thus obtained, encoding each region, can be connected directly or through a spacer to produce a polynucleotide encoding the CAR of the present invention. The connection of polynucleotides encoding each region can be conducted, as shown in the Examples described later, using known methods such as the overlap extension PCR method.

### <Vector Containing Polynucleotide Encoding Chimeric Antigen Receptor>

The present invention provides a vector containing the aforementioned polynucleotide. The vector of the present invention can be linear or circular, and examples thereof include viral vectors, plasmid vectors, episomal vectors, artificial chromosome vectors, and transposon vectors.

Examples of the viral vectors include retroviral vectors such as lentiviruses, Sendai virus vectors, adenovirus vectors, adeno-associated virus vectors, herpes virus vectors, vaccinia virus vectors, poxvirus vectors, poliovirus vectors, Sindbis virus vectors, rabies virus vectors, paramyxovirus vectors, and orthomyxovirus vectors.

As for the plasmid vectors, for example, animal cell expression plasmid vectors such as pcDNA3.1, pA1-11, pXT1, pRc/CMV, pRc/RSV, and pcDNAI/Neo are mentioned.

An episomal vector is a vector capable of autonomous replication outside the chromosome. Specific methods using episomal vectors are known (see Yu et al., Science, 2009, 324, pp. 797 to 801). Examples of the episomal vectors include vectors containing sequences necessary for autonomous replication derived from EBV, SV40, and the like as vector elements. As vector elements necessary for autonomous replication, specifically, they are the replication origin and the gene encoding proteins that bind to the replication origin to control replication, for example, in EBV, the replication origin oriP and EBNA-1 gene, and in SV40, the replication origin ori and SV40LT gene are mentioned.

Examples of the artificial chromosome vectors include YAC (Yeast artificial chromosome) vectors, BAC (Bacterial artificial chromosome) vectors, and PAC (P1-derived artificial chromosome) vectors.

In these vectors, even in cells with slow proliferation, genes can be introduced with high efficiency. Additionally, from the viewpoint of easy establishment of stably expressing strains, retroviral vectors are preferable.

The vector of the present invention may contain, in addition to the polynucleotide encoding the aforementioned CAR, expression control sequences such as promoters, enhancers, poly(A) addition signals, and terminators, replication origins as well as nucleotide sequences encoding proteins that bind to replication origins to control replication, and nucleotide sequences encoding other proteins.

By operably placing the polynucleotide encoding the aforementioned CAR or the nucleotide encoding other proteins described later downstream of the promoter, it becomes possible to efficiently transcribe each polynucleotide. Examples of such "promoters" include the CMV (cytomegalovirus) promoter, SRα promoter, SV40 early promoter, retroviral LTR, RSV (Rous sarcoma virus) promoter, HSV-TK (herpes simplex virus thymidine kinase) promoter, EF1α promoter, metallothionein promoter, heat shock promoter, and others.

Examples of "nucleotides encoding other proteins" include genes encoding fluorescent protein genes, reporter genes, drug resistance genes and other marker genes, and molecules involved in T-cell activation such as cytokines. Additionally, EGFR without an intracellular domain (truncated EGFR, tEGFR), as shown in the examples mentioned later, can be mentioned. Furthermore, by using DNA encoding the IRES, 2A peptide sequence (such as 2A peptide derived from Thosea asigna (T2A)) or the like, it becomes possible to polycistronically express the other proteins together with the aforementioned CAR.

Furthermore, in the body of the subject (such as a patient with a blood cancer) to whom the cells expressing CAR have been administered, to appropriately induce apoptosis of the administered CAR-expressing cells, the vector of the present invention may contain a "nucleotide encoding other proteins" as a suicide gene. Examples of suicide genes include herpes simplex virus thymidine kinase (HSV-TK) and inducible caspase 9 (iCasp9). Drugs that activate the function of such genes include ganciclovir for HSV-TK and AP1903, a chemical inducer of dimerization (CID), for iCasp9 (see Cooper LJ. et al., Cytotherapy. 2006; 8(2): pp. 105 to 17, Jensen M. C. et al., Biol Blood Marrow Transplant. 2010 Sep; 16(9): pp. 1245 to 56, Jones BS., Front Pharmacol. 2014 Nov 27; 5: 254., Minagawa K., Pharmaceuticals (Basel). 2015 May 8; 8(2): pp. 230 to 49, Bole-Richard E., Front Pharmacol. 2015 Aug 25; 6: 174).

### <Cells Expressing Chimeric Antigen Receptor>

The present invention provides cells expressing the CAR of the present invention. The cells of the present invention can be obtained by introducing a polynucleotide or vector encoding the aforementioned CAR of the present invention into the cells.

The "cell" into which the polynucleotide or vector of the present invention is introduced is preferably of mammalian origin, such as cells derived from humans, or cells derived from non-human mammals such as rodents (such as mice and rats), cows, sheep, horses, dogs, pigs, and monkeys. The type of cell is not particularly limited, and it is possible to use body fluids such as blood and bone marrow, or from tissues such as spleen, thymus, and lymph nodes, or cells collected from tumors or malignant ascites. A preferable example is immune cells used as cells for expressing CAR.

Examples of "immune cells" include CD4 positive CD8 negative T cells, CD4 negative CD8 positive T cells, T cells, αβ-T cells, γδ-T cells, NK cells, and NKT cells. Such immune cells include cells prepared from pluripotent stem cells such as iPS cells. If they include the aforementioned lymphocytes or precursor cells, various cell groups can be used. That is, PBMC (peripheral blood mononuclear cells) collected from peripheral blood can also be cited as an example of immune cells.

Before introducing the polynucleotide of the present invention, the cells for CAR expression may be activated. For example, they can be stimulated with anti-CD3 antibody and anti-CD28 antibody to activate the cells for CAR expression. More specifically, by culturing in a culture container (such as a culture dish) coated with anti-CD3 antibody and anti-CD28 antibody on the culture surface, stimulation can be added by the anti-CD3 antibody and anti-CD28 antibody. It is also possible to perform this stimulation using magnetic beads coated with anti-CD3 antibody and anti-CD28 antibody (for example, Dynabeads T-Activator CD3/CD28 provided by VERITAS).

Furthermore, as shown in the following Examples, to prevent exhaustion of the cells of the present invention, a tyrosine kinase inhibitor may be added to the medium during the production of these cells. An example of such a tyrosine kinase inhibitor is dasatinib.

To increase the survival rate/proliferation rate of cells, a culture fluid supplemented with T-cell growth factor may be used during activation treatment. IL-2, IL-15, IL-7, and the like can be used as T-cell growth factors. T-cell growth factors such as IL-2, IL-15, IL-7, and the like can be prepared according to usual methods. Commercially available products can also be used. Although the use of T-cell growth factors from non-human animal species is not excluded, usually, T-cell growth factors derived from humans (even if they are recombinant) are used.

Typically, for application thereof (administration to a subject), cells after the introduction of the polynucleotide of the present invention are proliferated. For example, cells introduced with the polynucleotide of the present invention are cultured using a culture fluid supplemented with T-cell growth factor (and subcultured if necessary). In addition to this culturing, it is possible to perform a similar treatment (reactivation) as in the case of activating CAR-expressing cells.

Furthermore, in the culturing of the cells of the present invention, a medium supplemented with serum (such as human serum or fetal bovine serum) may be used. However, by employing a serum-free medium, it is possible to prepare cells that have advantages of higher safety when applied clinically and less likeliness to show differences in culture efficiency due to differences between serum lots. When using serum, it is preferable to use autologous serum, that is, serum collected from the subject who is to receive the administration of the cells of the present invention.

Moreover, the cells of the present invention may be cells in which the function of proteins that suppress excessive immune responses and the like (such as immune checkpoint substances) is inhibited. Examples of such proteins include Programmed Death 1 (PD-1), PD-L1, PD-L2, CTLA-4, TIM-3, CEACAM (such as CEACAM-1, CEACAM-3, and CEACAM-5), LAG-3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, and GAL9.

Moreover, the cell of the present invention may be a cell in which the function of endogenous TCR, HLA, and other proteins is inhibited to facilitate allogeneic transplantation.

As a method for inhibiting the functions of a protein, there is a knockout method targeting a gene encoding the aforementioned protein, a method using dsRNA (double-stranded RNA, for example, siRNA) complementary to the transcription products of the aforementioned gene, a method using antisense RNA complementary to the transcription products, and a method using RNA with ribozyme activity that specifically cleaves the aforementioned transcription products. Also, genome editing methods that modify the target gene using site-specific nucleases (for example, zinc finger nucleases (ZFN), transcription activator-like effector nucleases (TALEN), DNA double-strand breaking enzymes such as CRISPR-Cas9) are suitably used to inhibit the functions of the aforementioned substances.

As a method for introducing the polynucleotide or vector of the present invention into a cell, examples thereof include methods such as lipofection, microinjection, calcium phosphate method, DEAE-dextran method, electroporation, and particle gun method. Also, if the vector of the present invention is a retroviral vector, an appropriate packaging cell can be selected based on the LTR sequence and packaging signal sequence possessed by that vector, and retrovirus particles can be prepared using this. Examples of packaging cells include PG13, PA317, GP+E-86, GP+envAm-12, and Psi-Crip. In addition, 293 cells and 293T cells with high transfection efficiency can be used as packaging cells.

Moreover, after introducing the polynucleotide or vector of the present invention into a cell, the expression of CAR in that cell can be confirmed by known methods such as flow cytometry, RT-PCR, Northern blotting, Western blotting, ELISA, and fluorescent immunostaining.

### <Multispecific Antibody>

As shown in the Examples described later, the target of the antibody of the present invention, HLA-DR, becomes a cell surface antigen specific to a blood cancer. Therefore, if it is possible to induce immune cells to blood cancer cells using this antigen as a marker and achieve cellular damage or the like, it can contribute to the treatment of the disease. As a mode enabling such immunotherapy, a multispecific antibody is mentioned.

A "multispecific antibody" means a monoclonal antibody that has multiple antigen recognition sites and has binding specificity to at least two different antigens.

In the present invention, the multispecific antibody has the antibody of the present invention or a functional fragment thereof and the antigen recognition site for immune cells. Such immune cells are not particularly limited, and are as exemplified in the aforementioned <Cells Expressing Chimeric Antigen Receptor>, but from the viewpoint of being able to inflict cellular damage or the like on target cells such as blood cancer cells, effector cells are preferable. Examples of effector cells include NK (natural killer) cells, monocytes, macrophages, and eosinophils. Further, antigens that the multispecific antibody of the present invention can recognize on such cells include stimulatory or inhibitory receptors present on the surface of the cells. More specific examples thereof include CD3, CD16, B7-H4, BTLA, CD4, CD8, CD25, CD27, CD28, CD32, CD56, CD137, CTLA-4, GITR, HVEM, ICOS, LAG-3, NKG2D, OX40, PD-1, TIGIT, TIM-3, VISTA, 4-1BB, NK-expressed KIR, B7-1, B7-2, B7H3, PD-L1, PD-L2, and TNFSF9.

A preferable embodiment of the multispecific antibody of the present invention is a bispecific antibody, and more specific examples thereof include BsDb (bispecific diabody), scBsDb (single-chain bispecific diabody), scBsTafv (single-chain bispecific tandem variable domain), DNL-(Fab)3 (dock-and-lock trivalent Fab), sdAb (single domain antibody), BssdAb (bispecific single-stranded antibody complex), knobs-into-holes BsAb IgG, Ig-scFv fusion type, diabody-Fc fusion type, dual variable domain antibody (DVD-IgG).

Further, such bispecific antibodies can be produced by those skilled in the art using appropriate known methods. Examples of such known methods include methods of production from a further fusion cell between a hybridoma that produces an antibody and the like, recognizing HLA-DR, and a hybridoma that produces an antibody and the like, recognizing an immune cell (quadroma method). Example methods also include a method for chemically linking functional fragments of these different antibodies (such as Fab). Example methods further include a method that fuses the DNA encoding functional fragments of these different antibodies through a linker, introduces the resulting DNA construct into a host cell, and produces the target bispecific antibody from the cell. Moreover, example methods include methods that promote heterodimerization of different antibodies or functional fragments thereof, such as Knob-into-Hole (Genentech), Triomab/Quadroma (Trion Pharma/Fresenius Biotech), CrossMAb (Roche), electrostatic-matched (Amgen), LUZ-Y (Genentech), Strand Exchange Engineered Domain body (SEEDbody) (EMD Serono), Biclonic (Merus), and Duo Body (GenmabA/S), and controlled Fab-ARM exchange method (CFAF method) described in International Publication No. WO2008/119353 and International Publication No. WO2011/131746, and the method described in Chengbin Wu et al., Generation and Characterization of a Dual Variable Domain Immunoglobulin (DVD-Ig™) Molecule, Springer Nature Experiments, 2010.

### <Antibody-Drug Conjugate>

As shown in the Examples described later, the target of the antibody of the present invention, HLA-DR, becomes a cell surface antigen specific to a blood cancer. Therefore, the present invention may also encompass an embodiment of an antibody-drug conjugate (ADC) formed by combining the antibody of the present invention (particularly humanized antibodies) or a functional fragment thereof with a drug.

In the ADC of the present invention, the "drug" that can be combined with the antibody is not particularly limited, and examples thereof include cytotoxic substances known in the art. Examples of "cytotoxic substances" include anticancer agents such as irinotecan (CPT-11), irinotecan metabolite SN-38 (10-hydroxy-7-ethylcamptothecin), adriamycin, taxol, 5-fluorouracil, nimustine, ranimustine, temozolomide, and other alkylating agents, gemcitabine, hydroxycarbamide, and other metabolic antagonists, etoposide, vincristine, and other plant alkaloids, mitomycin, bleomycin, and other anticancer antibiotics, cisplatin and other platinum drugs, sorafenib, erlotinib, and other targeted molecular agents, methotrexate, cytosine arabinoside, 6-thioguanine, 6-mercaptopurine, cyclophosphamide, ifosfamide, busulfan, MMAE (monomethyl auristatin E), DM1 (mertansine), and calicheamicin. Examples of cytotoxic substances also include radioactive isotopes such as ³²P, ¹⁴C, ¹²⁵I, ³H, ¹³¹I, ¹⁸⁶Re, ¹⁸⁸Re, ¹⁰B, ¹¹¹In, and ⁹⁰Y. The cytotoxic substances may be light-sensitive materials that exert cytotoxic activity, specifically, substances that, when activated by light irradiation, change to a form that exhibits cytotoxicity (cell toxicity) themselves, or substances that cause production of cytotoxic substances (cell toxicity substances). Examples thereof include chlorins, chlorine e6, porfimer sodium, talaporfin sodium, verteporfin, and precursors and derivatives thereof. Additionally, cytotoxic substances include toxic peptides, such as saporin, ricin, and ribosome-inactivating proteins (RIPs) such as Shiga toxin.

Furthermore, the binding between the antibody and the cytotoxic substance can be carried out using known methods in the art, and both direct and indirect binding are acceptable. For direct binding, covalent binding can be used. For indirect binding, binding via a linker can be utilized. General techniques regarding linkers are described, for example, in Hermanson, G.T. Bioconjugate Techniques, Academic Press, 1996; Harris, J. M. and Zalipsky, S. (Eds.), Poly(ethylene glycol), Chemistry and Biological Applications, ACS Symposium Series, 1997; Veronese, F. and Harris, J. M. (Eds.), Peptide and protein PEGylation. Advanced Drug Delivery Review 54(4), 2002. Also, the linker can be a linear linker (divalent linker) or a branched linker (trivalent or higher linker).

### <Pharmaceutical Composition>

The present invention pertains to a pharmaceutical composition containing, as its active ingredient, an antibody of the present invention or a variable region thereof, or an antibody construct that has it (cells expressing the aforementioned chimeric antigen receptor, multispecific antibodies, antibody-drug conjugates (ADC), and the like) (hereinafter simply referred to as "antibody or the like of the present invention"). The present invention also provides a method for treating diseases (for example, blood cancers such as acute myeloid leukemia) that involves administering a therapeutic effective amount of the antibody or the like of the present invention to a subject.

The "diseases" targeted by the present invention are not particularly limited as long as they are diseases related to cells that have HLA-DR as a specific surface antigen, and examples thereof include a blood cancer. More specific examples thereof include acute myeloid leukemia and related diseases thereof (myelodysplastic syndrome (MDS), acute lymphoblastic leukemia (ALL), B-cell malignant lymphoma, and multiple myeloma) . "Blood cancers" can be primary cancers, recurrent cancers, secondary cancers, or metastatic cancers. "Acute myeloid leukemia (AML)" refers to a diverse blood tumor characterized by the clonal autonomous proliferation of immature myeloid lineage cells whose differentiation and maturation are impaired. It is also a cancer of the myeloid lineage of blood cells, which is characterized by the rapid proliferation of abnormal leukemia cells in the bone marrow, and as a result, the normal hematopoietic function is significantly inhibited. This leads to symptoms such as decreased white blood cells, anemia, and thrombocytopenia.

The "pharmaceutical composition" of the present invention can contain, in addition to the antibody or the like of the present invention, additional pharmaceutically acceptable ingredients. Examples of such additional ingredients include carriers, emulsifiers, wetting agents, pH buffering agents, media, excipients, disintegrants, buffering agents, isotonic agents, suspending agents, solubilizing agents, soothing agents, stabilizers, preservatives, and antiseptics. More specifically, other additional pharmaceutically acceptable ingredients can include, for liquid preparations like injections, aqueous solutions (such as physiological saline, water for injection, phosphate-buffered saline, glucose solutions, and glycerol solutions), and aluminum hydroxide, for example. For freeze-dried preparations, examples include, but are not limited to, sugars (such as mannitol, lactose, and sucrose) and albumin. Furthermore, the pharmaceutical composition of the present invention can be in the form of a kit in which the above components can be mixed prior to administration. In the case of use as an injection, it can also be in the form of an injection introduced into a syringe.

The pharmaceutical composition of the present invention may contain only the antibody or the like of the present invention as an active ingredient, or it may contain the antibody or the like and at least one different anticancer agent. Also, the antibody or the like of the present invention can be administered in conjunction with other anticancer agents, thereby enhancing the antitumor effect. Other anticancer agents used for such purposes can be administered to the subject simultaneously with the antibody or the like of the present invention, separately, or consecutively, and the dosing interval can be adjusted. Examples of such "anticancer agents" include venetoclax, azacitidine, dasatinib, cytarabine (Ara-C, Cytosar-U), enocitabine, quizartinib (AC220), sorafenib (BAY 43-906), lestaurtinib (CEP-701), midostaurin (PKC412), carboplatin, carmustine, chlorambucil, dacarbazine, ifosfamide, lomustine, mechlorethamine, procarbazine, pentostatin, (2'-deoxycoformycin), etoposide, teniposide, topotecan, vinblastine, vincristine, paclitaxel, dexamethasone, methylprednisolone, prednisone, all-trans retinoic acid, arsenic trioxide, interferon-α, rituximab (Rituxan (registered trademark)), gemtuzumab ozogamicin, imatinib mesylate, Cytosar-U, melphalan, busulfan (Myleran (registered trademark)), thiotepa, bleomycin, platinum (cisplatin), cyclophosphamide (Cytoxan (registered trademark)), daunorubicin, doxorubicin, idarubicin, mitoxantrone, 5-azacytidine, cladribine, fludarabine, hydroxyurea, 6-mercaptopurine, methotrexate, 6-thioguanine, immune checkpoint inhibitors (such as anti-PD-1 antibodies), or any combination thereof, but it is not limited to agents with antitumor activity. Additionally, the pharmaceutical composition of the present invention and the antibody or the like of the present invention can be combined with other cancer treatment methods such as radiation therapy and cancer immunotherapy, not limited to chemotherapy using the aforementioned anticancer agents.

Furthermore, in the case of a pharmaceutical composition containing the cells of the present invention, it may contain other ingredients such as dimethyl sulfoxide (DMSO) and serum albumin for the purpose of protecting the cells, and antibiotics for the purpose of preventing bacterial contamination. Furthermore, it may contain cytokines, growth factors, steroids and other T-cell activating factors, vitamins, immunostimulants, and immune checkpoint inhibitors for the purpose of activating, proliferating, or inducing differentiation of cells, and the cells of the present invention can be used in conjunction with these other ingredients.

The administration method of the pharmaceutical composition varies depending on the type of substance to be administered, the form of the composition, the age, weight, gender, and health condition of the subject, but it can be administered either by parenteral administration (such as intravenous administration, arterial administration, or local administration) or by oral administration. The preferable method of administration is parenteral, and more preferably, intravenous. Also, without systemic administration, local administration may be performed. As local administration, direct injection into the targeted tissue (such as bone marrow) can be exemplified.

The dose of the pharmaceutical composition can vary depending on the age, weight, gender, and health condition of the subject, the progression of the symptoms, and the ingredients of the pharmaceutical composition being administered. Also, the dosing schedule can be adjusted appropriately based on these conditions, and in addition to a single dose, it can be administered multiple times continuously or periodically, and generally, when administering the antibody intravenously, for adults, it is 0.1 to 1000 mg per 1 kg of body weight per day, preferably 1 to 100 mg. Also, when administering ADC, in terms of the drug amount contained, it is 0.001 to 1000 mg per 1 kg of body weight per day for adults, preferably 0.01 to 100 mg. The number of times these antibodies or ADC are administered can be, for example, once, or multiple times at intervals of 1 day to 1 year (for example, every week, every 10 to 30 days, monthly, every 3 to 6 months, every six months, or annually).

Also, as for dose of the cell (for example, the cell expressing the chimeric antigen receptor) used in the treatment method of the present invention , in one administration, for adults, it can be set at 1 x 10³ to 1 x 10¹⁰ cells per 1 kg of body weight, preferably 1 x 10⁴ to 1 x 10⁹ cells, and more preferably, 1 x 10⁵ to 1 x 10⁸ cells. The dosing interval can be, for example, every week, every 10 to 30 days, monthly, every 3 to 6 months, annually, or the like. Also, the cells of the present invention can proliferate autonomously within the body of the administration subject, so that it can also be a single dose. Additionally, after administration, the number of cells of the present invention in the body can be monitored, and the dosing timing can be determined based on the results.

In the present invention, as the "subject" to be administered with the antibody or the like of the present invention, for example, humans suffering from a blood cancer, humans who might relapse with a blood cancer, and humans who have relapsed with a blood cancer can be mentioned. In the present invention, "treatment" refers to alleviating symptoms or accompanying symptoms characteristic of a blood cancer and the like (mild symptoms), preventing or delaying the exacerbation of symptoms, and improving clinical test value abnormalities such as the increase in blood cancer cells. Furthermore, the prevention, delay, or reduction of the risk in relapse of a blood cancer is also included.

In particular, antibody or the like of the present invention can show allele specificity to HLA-DR. For blood cancer patients and the like with relapse after HLA-DR mismatched allogeneic transplantation, if the HLA-DR of the recipient (such as blood cancer patient) is a positive type that the antibody or the like of the present invention binds to, and the HLA-DR type of the donor is a negative type that the antibody of the present invention does not bind to, then CAR-T cells prepared from the patient themselves who received the transplant or from donor-derived cells can specifically attack the blood cancer of the recipient.

Furthermore, the antibody of the present invention binds only to some normal cells, such as B cells, in normal blood cells and does not bind to monocytes and the like. Moreover, it only binds to a part of hematopoietic stem cells, which are the source of all blood cells. Thus, even if regular autologous CAR-T cells and the like are used for treatment in blood cancer patients and the like with the positive HLA-DR that the antibody or the like of the present invention binds to, blood cancer cells are eliminated, normal hematopoiesis is maintained by negative cells that the antibody or the like of the present invention does not bind to, making treatment possible.

### [Examples]

Hereinafter, the present invention will be described in more detail based on Examples, but the present invention is not limited to the following Examples. The Examples were carried out using the materials and methods shown below.

### (Patient Specimen)

Bone marrow derived from acute myeloid leukemia (AML) patients was collected from the iliac bone after obtaining consent. Mononuclear cells were separated using Ficoll Paque (GE Healthcare) and used for analysis.

### (Preparation of Anti-AML Monoclonal Antibody)

BALB/c mice aged 6 weeks (CLEA Japan) were immunized by injecting AML cell lines (KG1a, THP-1, U937, HL60, and MOLM-13) into their footpads. After four rounds of immunization, lymphocytes harvested from the popliteal lymph nodes and SP2/0 mouse myeloma cells were cell-fused using PEG 1500 (Roche Applied Science), and cells were then seeded into a 96-well plate in HAT medium to select hybridomas. To identify hybridomas that produce monoclonal antibodies that bind to AML cells and do not bind to normal blood cells, firstly, AML cell lines were reacted with monoclonal antibodies in the supernatant of each hybridoma, and after that, they were reacted with PE-labeled anti-mouse IgG antibody (BioLegend 405307) and analyzed by flow cytometry. Then, peripheral blood mononuclear cells (PBMC) derived from healthy individuals were stained similarly, and after selecting hybridomas producing monoclonal antibodies that did not bind to normal blood cells, the cells and supernatant were stored and used for subsequent analysis.

### (Flow Cytometry Analysis)

Single cell suspensions were stained with fluorescent-labeled antibodies. For staining, the following were used: anti-CD34-APC (8G12, BD Biosciences), anti-CD38-FITC (HIT2, BioLegend), anti-CD3-Cy7PE (UCHT1, BioLegend), anti-CD19-Cy7APC (HIB19, BioLegend), anti-CD14-BV510 (M5E2, BioLegend), anti-HLA-DR-APC (L243, BioLegend), anti-CD11c-FITC (Bu15, BioLegend), anti-CD19-PE (HIB19, BioLegend), anti-CD279-BV510 (EH12.2H7, BioLegend), anti-CD223-FITC (11C3C65, BioLegend), anti-CD366-PE (F38-2E2, BioLegend), goat anti-mouse IgG-PE (405307, BioLegend), goat anti-mouse F(ab')2-Alexa Fluor 647 (115-605-072, Jackson ImmunoResearch), Biotin-conjugated anti-EGFR antibody (Erbitux), Streptavidin-PE (405204, BioLegend). KG2032 (mouse IgG2a) was purified using Protein G Sepharose 4 Fast Flow (GE Healthcare) and used for staining at a concentration of 10 µg/ml. Analysis and cell sorting were performed using FACSCanto II or FACSAria II (BD Biosciences). Data was analyzed using FlowJo software. For the analysis using activated T cells, PBMCs derived from healthy individuals were stimulated at 1 × 10⁶/ml with 3 µg/ml of PHA for three days.

### (Identification of KG2032 Antigen Using CRISPR gRNA Library)

First, clones of Daudi cells expressing the Cas9 protein were obtained. Specifically, Daudi cells were first infected with the Cas9-expressing lentivirus vector, and after adding 10 µg/ml of blasticidin for 4 days to select cells where the vector was introduced, single-cell sorting was performed on a 96-well plate. After amplifying these cells, the Cas9 reporter (see Tzelepis K et al., Cell Rep. 2016; 17(4): 1193-205) was introduced to select clones with good knockout efficiency.

Next, to these resulting clones of Daudi cells expressing the Cas9 protein, the Human Genome-wide Knockout CRISPR Library (provided by the Yusa Lab of Kyoto University) was infected to achieve approximately a 30% introduction efficiency. After that, 0.5 µg/ml of puromycin was added for 3 days to select cells into which the CRISPR library was introduced. These cells were further expanded, stained with 2 × 10⁸ cells with KG2032, and 5 × 10⁶ cells were sorted from the fraction with weak KG2032 binding (KG2032low). DNA was extracted from the cells after sorting using the DNeasy Blood & Tissue Kits (QIAGEN), and NGS analysis was performed. DNA was also extracted from 5 × 10⁷ cells before sorting using the Blood & Cell Culture DNA Maxi Kit (QIAGEN) and used as a control.

### (Preparation of Knockout Cells)

For the synthesis of guide RNA (gRNA) to knockout HLA-DR, the GeneArt Precision gRNA Synthesis Kit (Thermo Fisher Scientific) was used. The following two types of gRNA were synthesized:
gRNA sequence (1): GTGCGCTTCGACAGCGACGT (SEQ ID NO: 31), and
gRNA sequence (2): GACGGAGCGGGTGCGGTTCC (SEQ ID NO: 32).

2 × 10⁵ Daudi cells were genetically introduced with 1 µg of TrueCut Cas9 Protein v2 (Thermo Fisher Scientific) and 200 ng of the prepared gRNA using the NEON Transfection System (Invitrogen). Nine days later, they were stained with anti-HLA-DR-APC, and cells with knocked-out HLA-DR were sorted and used for analysis.

### (Identification of Epitope of KG2032)

Five chimeras of HLA-DRB1*14:54 (KG2032-positive) and HLA-DRB1*1502 (KG2032-negative) were constructed by overlapping PCR. The constructed chimeras were introduced into K562 cells using a retroviral vector, and two days later, they were stained with KG2032 and L243, and the presence or absence of binding was analyzed by flow cytometry.

### (Construction and Preparation of Chimeric Antigen Receptor (CAR) 1)

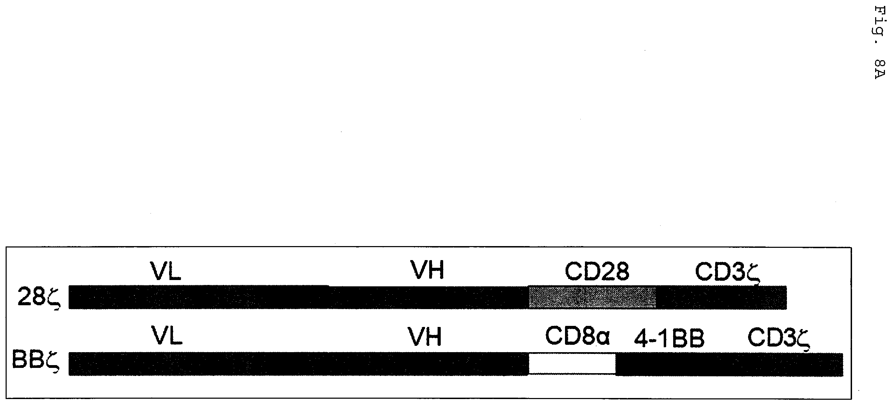

cDNA encoding the variable part of KG2032 was obtained using the SMARTer RACE 5'/3' Kit (Takara) and the 5' RACE method, and its sequence was identified. As shown in FIG. 8A, the light chain (VL) and heavy chain (VH) were fused using overlapping PCR, with a linker sequence in between. Furthermore, this, along with CD28 and CD3ζ, or CD8α, 4-1BB, and CD3ζ, was fused by overlapping PCR (referred to in FIG. 8A as "28ζ" and "BBζ") and inserted into a retroviral vector to construct the KG2032-CAR expression vector (also referred to as "KG2032-CD28-CD3ζ-CAR" and "KG2032-BBz-CAR" respectively). To allow the introduction efficiency to be measured with an anti-EGFR antibody, a sequence of EGFR lacking the intracellular domain was added to the KG2032-CAR expression vector after the CD3ζ in a manner that follows the T2A sequence.

### (Preparation of CAR-T Cells)

After introducing each KG2032 CAR expression vector into 293T cells using Lipofectamine 2000 (Invitrogen) along with the Gag-pol vector and VSV-G vector, the supernatant was collected 48 hours and 72 hours later to prepare the virus vector. PBMCs derived from healthy individuals were stimulated with anti-CD3 antibody (OKT3, eBioscience) and anti-CD28 antibody (CD28.2, eBioscience) to prepare activated T cells, which were then infected with the virus-containing supernatant. Afterward, the T cells into which CAR was introduced were cultured in a medium supplemented with 100 IU/ml of IL-2 for 6 days. When adding dasatinib, it was introduced at a concentration of 1 µM on days 4 to 6. On day 6, the introduction efficiency of CAR was analyzed using goat anti-mouse F(ab')2-Alexa Fluor 647 (115-605-072, Jackson ImmunoResearch) or biotin-conjugated anti-EGFR antibody.

### (Measurement of Cytokine Production 1)

The cytokine production of KG2032 CAR-T cells or control T cells was measured using the Human IFN-gamma Quantikine ELISA Kit/Human IL-2 Quantikine ELISA Kit (R&D Systems). Specifically, effector cells and target cells were cocultured in a 96-well plate, each at 1 × 10⁵ cells, and the supernatant collected after 16-24 hours was diluted to fit within the range of the standard curve for measurement.

### (Measurement of Cytotoxic Activity 1)

The tumor cell damaging ability of T cells was measured by the ⁵¹Cr release assay. 5 × 10⁵ target cells were added with 25 µ Ci of sodium chromate ⁵¹Cr solution (PerkinElmer), incubated at 37°C for 1.5 hours, and then cocultured with labeled target cells (1 × 10⁴ cells) and effector cells at various Effector/Target ratios. The ⁵¹Cr released into the supernatant after 4 hours was counted with a gamma counter. The ⁵¹Cr released either totally or spontaneously was measured by culturing 1 × 10⁴ labeled cells with 1% Triton X-100 or in medium. The cytotoxic activity was calculated as [(specific ⁵¹Cr release - spontaneous ⁵¹Cr release) / (total ⁵¹Cr release - spontaneous ⁵¹Cr release)] × 100.

### (Xenograft Mouse Model 1)

KG1a-luc/venus was produced by introducing the luciferase gene into KG1a cells using a lentivirus vector. Female NOD/SCID/IL-2Rγnull (NOG) mice (Japan CLEA), aged 6 to 8 weeks, were irradiated with 1.2 Gy of radiation, and then 4 × 10⁶ luciferase-expressing KG1a cells (KG1a-luc/venus) were injected via the tail vein. Five days later, VivoGlo Luciferin (Promega, 150 mg/kg body weight) was administered intraperitoneally, images were captured by the in vivo imaging system (IVIS), and analyzed using Living Image software (PerkinElmer). The next day, 1-2 × 10⁶ CAR-T cells or control T cells were injected intravenously, and IVIS imaging was performed weekly thereafter.

### (Construction and Preparation of Chimeric Antigen Receptor (CAR) 2)

cDNAs of the variable regions of the light chain and heavy chain of KG2032 were fused with CD28 and CD3ζ cDNAs by overlapping PCR. In addition, T2A-IL-15 cDNA was fused with the KG2032 CAR by overlapping PCR.

### (Preparation of CAR-NK Cells)

KG2032-non-reactive cord blood (CB) was identified by measuring KG2032 reactivity to CD19+ B cells by flow cytometry. T cells were removed using CD3 MicroBeads (Miltenyi Biotec). T-cell-depleted CB mononuclear cells were stimulated with 100 Gy-irradiated K562-mb15-41BBL cells and cultured in the presence of 20 IU/mL IL-2. After 1 week, retrovirus carrying KG2032 CAR-T2A-IL-15 cDNA was transduced into CB-derived NK cells using RetroNectin. The cells were then re-stimulated with 100 Gy-irradiated K562-mb15-41BBL cells, cultured for 1 additional week, and used for further experiments.

### (Measurement of Cytotoxic Activity 2)

The ability of NK cells to lyse tumor target cells was measured with ⁵¹Cr release assays. Briefly, 6 × 10⁵ target cells were labeled at 37°C for 1.5 h with 25 µCi of [⁵¹Cr] sodium chromate (PerkinElmer). Labeled target cells (1 × 10⁴) were incubated with effector cells for 4h. ⁵¹Cr release in harvested supernatants was counted with a gamma counter. Total and spontaneous ⁵¹Cr release was determined by incubation of 1 × 10⁴ labeled targets in either 1% Triton X-100 or culture medium. The cytotoxic Activity was calculated as ([specific ⁵¹Cr release - spontaneous ⁵¹Cr release] / [total ⁵¹Cr release - spontaneous ⁵¹Cr release]) × 100.

### (CD107a degranulation assay)

KG2032 CAR-NK cells or control NK cells (2.5 × 10⁵ cells) were co-cultured in 96-well plates with KG1a AML cells or BM cells from an AML patient (5 × 10⁴ cells) at an effector : target ratio of 5:1. Anti-CD107a Alexa Fluor 647 (1:50) and monensin (BD Biosciences, 1:1500) were added at the start of incubation. After incubation for 5h, cells were stained with anti-CD56-PE and analyzed by flow cytometry. Note that, an allele type of HLA-DR of the AML patient is DRB1*08:02/12:01.

### (Xenograft Mouse Model 2)

A disseminated AML model was established by intravenous transfer of KG1a AML cells into NOD/SCID/IL-2Rγnull (NOG) mice (In Vivo Science). Six-to eight-week-old female NOG mice (irradiated with 1.0-2.4Gy 4-24h before transplantation) were intravenously injected via the tail vein with 1-4 × 10⁶ KG1a-luc cells. In the experiments with KG2032 CAR-NK cells, 1.0-1.4 × 10⁶ KG2032 CAR-NK cells or control NK cells were infused on days 1 and 3 after the transfer of AML cells. Tumor growth in the mice was measured by measuring fluorescence upon administration of luciferin using IVIS.

### (Preparation of Modified KG2032 CAR-T Cells)

cDNAs of the variable regions of the light chain and heavy chain of KG2032 were fused with CD8α, 4-1BB, and CD3ζ cDNAs by overlapping PCR. Mutations for substituting tyrosine in the CD3z ITAM motif in the KG2032 CAR sequence with phenylalanine were introduced using PrimeSTAR Mutagenesis Basal Kit (Takara).

### (Preparation of Single-cell Suspensions of Mucosal Epithelial Cells)

To prepare single-cell suspensions of mucosal epithelial cells, mucous membranes were cut into 3-mm-thick pieces, washed with phosphate-buffered saline (PBS) (Nacalai Tesque) three times, and incubated in HBSS (Nacalai Tesque) containing 10 mM EDTA at 37°C for 30 min. Epithelial cells were then peeled off by shaking and collected. After centrifugation at 400 g for 5min, cells were suspended in TrypLE Express (Thermo Fisher Scientific) containing 500 U/mL DNase I and incubated at 37°C for 30 min. DNA was extracted from cells using the DNeasy Blood & Tissue Kit (QIAGEN). Typing of HLA-DRB1 was performed by the HLA Laboratory (Kyoto, Japan).

### (Measurement of Cytokine Production 2)

The modified KG2032 CAR-T cells or control T cells were cocultured with intestinal epithelial cells or Leukemia cells (1 × 10⁵ cells for each) for 16h. Cytokines secreted in the supernatant of cell cocultures were measured using ELISA kits (IFN-γ and IL-2; R&D Systems) .

### (Measurement of Cytotoxic Activity 3)

The cytotoxic ability of the modified KG2032 CAR-T cells was measured with ⁵¹Cr release assays. 6 × 10⁵ target cells were labeled at 37°C for 1.5h with 25 µCi of [⁵¹Cr]sodium chromate (PerkinElmer). Labeled target cells (1 × 10⁴) were incubated with effector cells for 4h. ⁵¹Cr release in harvested supernatants was counted with a gamma counter. Total and spontaneous ⁵¹Cr release was determined by incubation of 1 × 10⁴ labeled targets in either 1% Triton X-100 or culture medium. The cytotoxic activity was calculated as ([specific ⁵¹Cr release - spontaneous ⁵¹Cr release] / [total ⁵¹Cr release - spontaneous ⁵¹Cr release]) × 100.

### (Xenograft Mouse Model 3)

A disseminated AML model was established by intravenous transfer of KG1a AML cells into NOD/SCID/IL-2Rγnull (NOG) mice (In Vivo Science). Six-to eight-week-old female NOG mice (irradiated with 1.0-2.4Gy 4-24h before transplantation) were intravenously injected via the tail vein with 1-4 × 10⁶ KG1a-luc cells. On day 5, the mice were intraperitoneally infused with VivoGlo Luciferin (Promega, 150 mg/kg body weight), and tumor growth was measured by observing the fluorescence emitted by the tumor using an in vivo imaging system (IVIS) with Living Image software (PerkinElmer). The mice were then intravenously injected with 1.1-2.2 × 10⁶ the modified KG2032 CAR-T cells or control T cells.

### (Xenograft Mouse Model 4)

DNA was extracted from cells using the DNeasy Blood & Tissue Kit (QIAGEN). Typing of HLA-DRB1 was performed by the HLA Laboratory (Kyoto, Japan). AML cells carrying KG2032-reactive HLA-DRB1 were used in this experiment.

Intra-BM transplantation with BM cells from AML patients was performed. Six- to eight-week-old female NOG mice were irradiated with 1.2Gy 4-24h before transplantation, then injected in the left tibia with 4 × 10⁵ CD3-depleted AML cells. Six days later, the mice were intravenously injected with 2 × 10⁶ the modified KG2032 CAR-T cells or control T cells. One month after infusion, the BM cells were stained with anti-hCD45-Cy7APC(2D1, BioLegend), anti-hCD34-APC(8G12, BD Biosciences), anti-hCD3-PE(HIT3a, BioLegend), anti-mTer119-PerCPCy5.5(Ter-119, BioLegend), and anti-mCD45-Cy7PE(30-F11, BioLegend), and analyzed by flow cytometry.

The results of the experiments conducted using the above materials and methods are presented below.

### (Identification of AML-Specific Monoclonal Antibody KG2032)

With the aim of obtaining AML-specific monoclonal antibodies (mAb), approximately 14,000 hybridomas were first established that secrete monoclonal antibodies (mAb) binding to various AML cell lines and AML cells, derived from the bone marrow (BM) cells of AML patients.

Next, from these mAbs, 1,078 mAbs that do not bind to cells other than B cells in peripheral blood mononuclear cells (PBMCs) from healthy donors were selected. Further, AML patient BM cells were stained with these candidate mAbs, and the clone named KG2032 as the mAb specifically binding to AML was eventually identified.

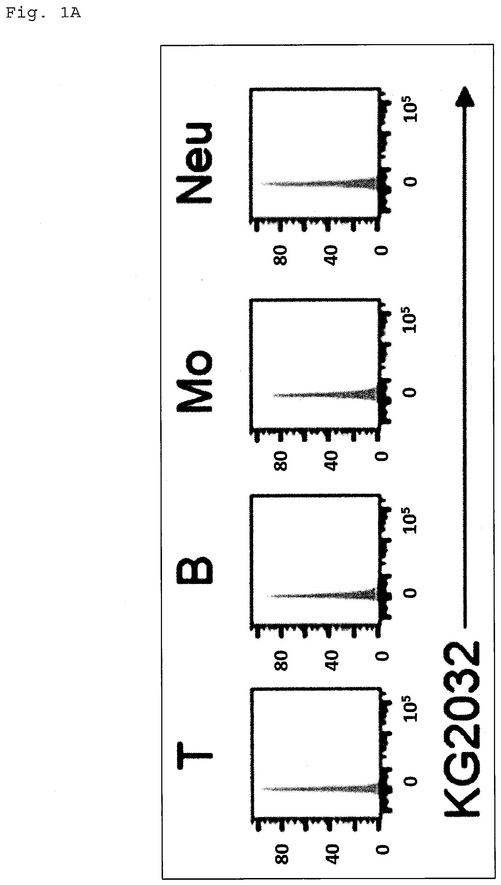

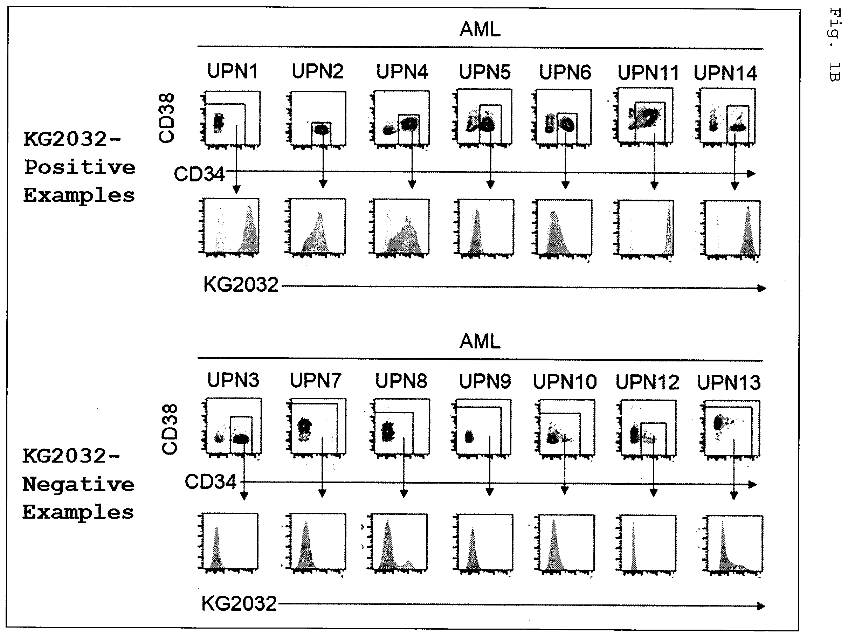

KG2032, as shown in FIG. 1A, did not show binding in certain PBMCs derived from a healthy donor used for screening. On the other hand, as shown in FIG. 1B, KG2032 bound to almost all leukemia cells in 7 out of 14 AML patients.

### (Identification of Antigen Recognized by KG2032)

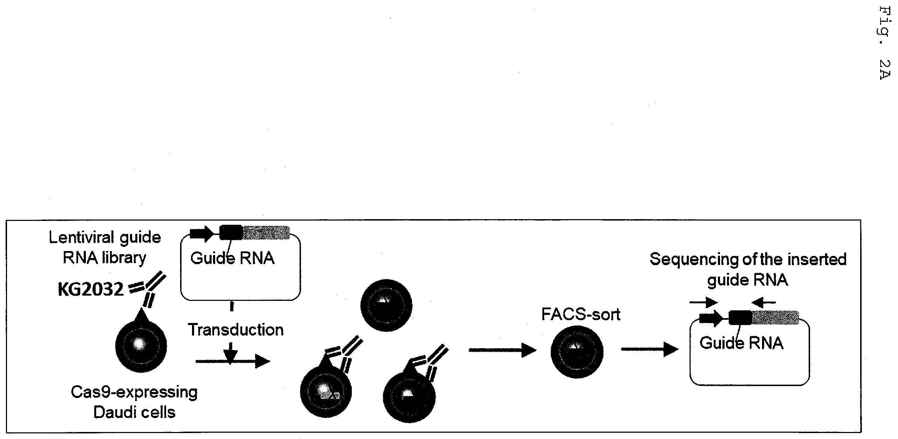

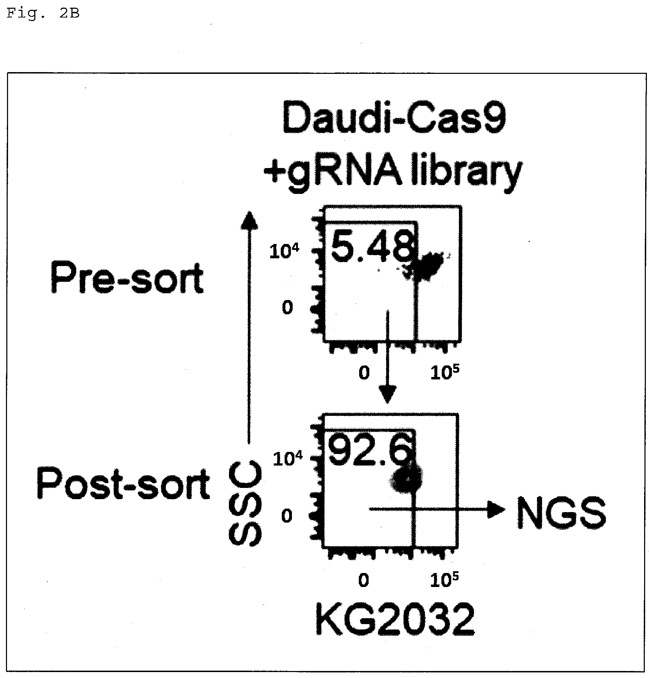

In the step shown in FIG. 2A, identification of the antigen recognized by KG2032 was endeavored. First, the Human Genome-wide Knockout CRISPR Library was introduced into Daudi cells expressing Cas9 protein and the cells were concentrated into which this library was introduced using puromycin. Then, these cells were expanded, 2 × 10⁸ cells were stained with KG2032, and 5 × 10⁶ fractions of KG2032low 5% were sorted (FIG. 2B).

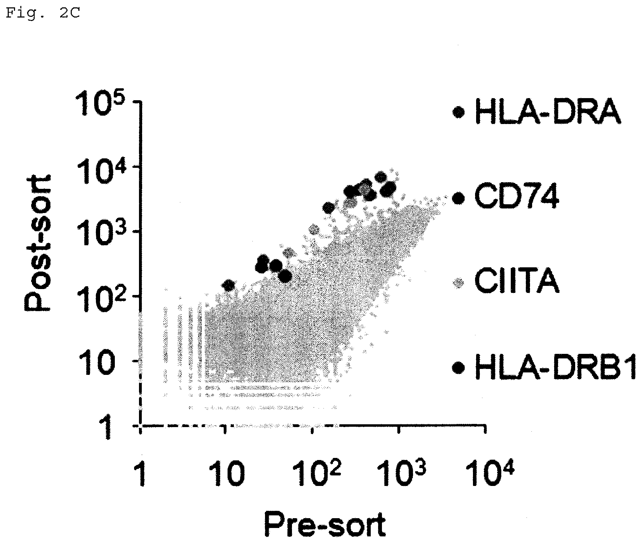

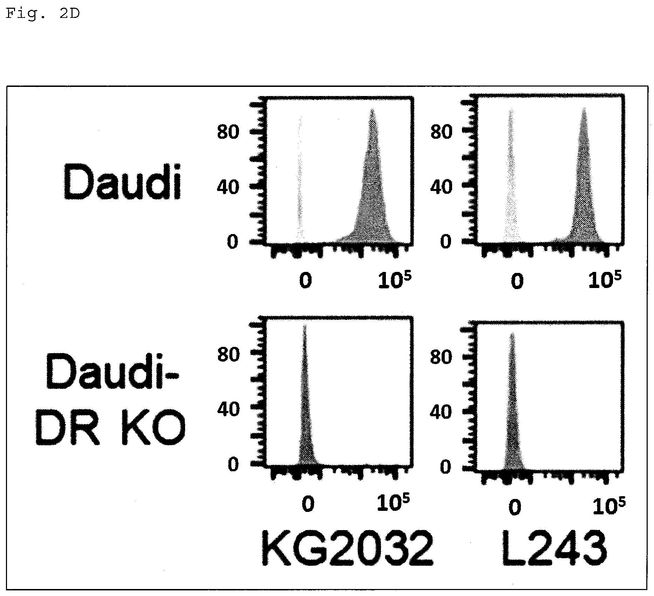

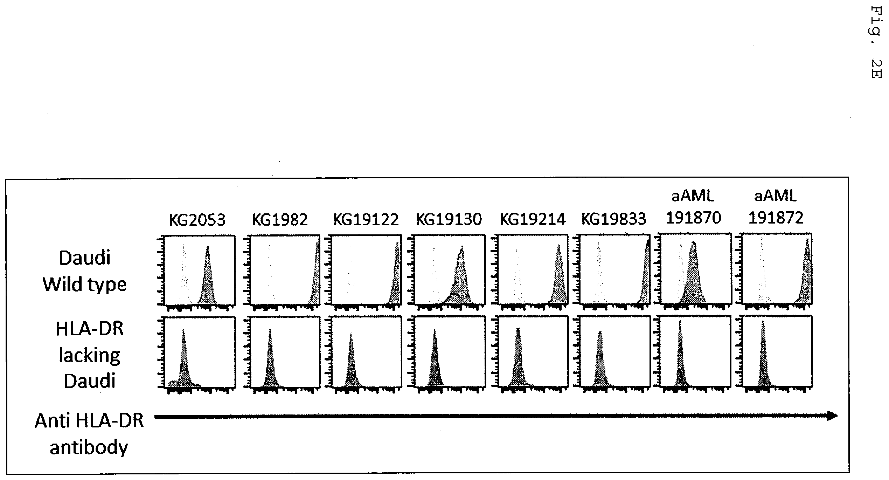

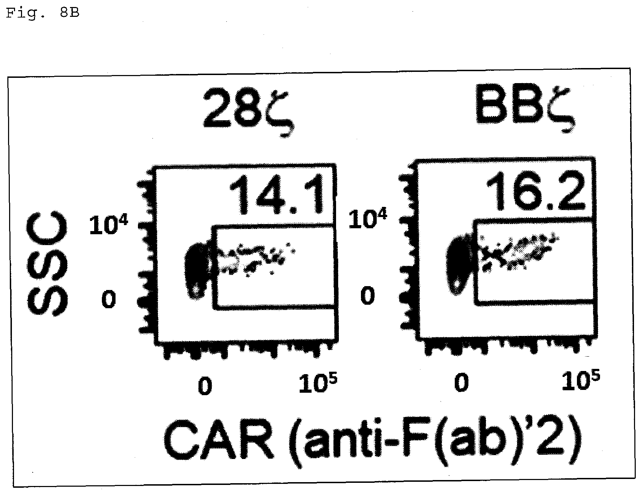

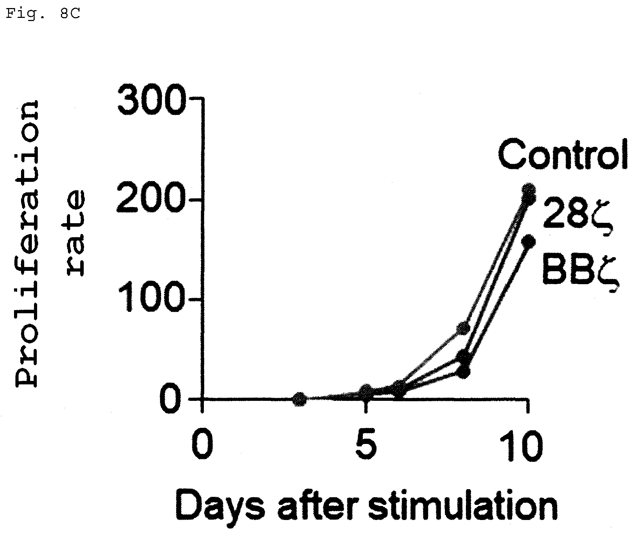

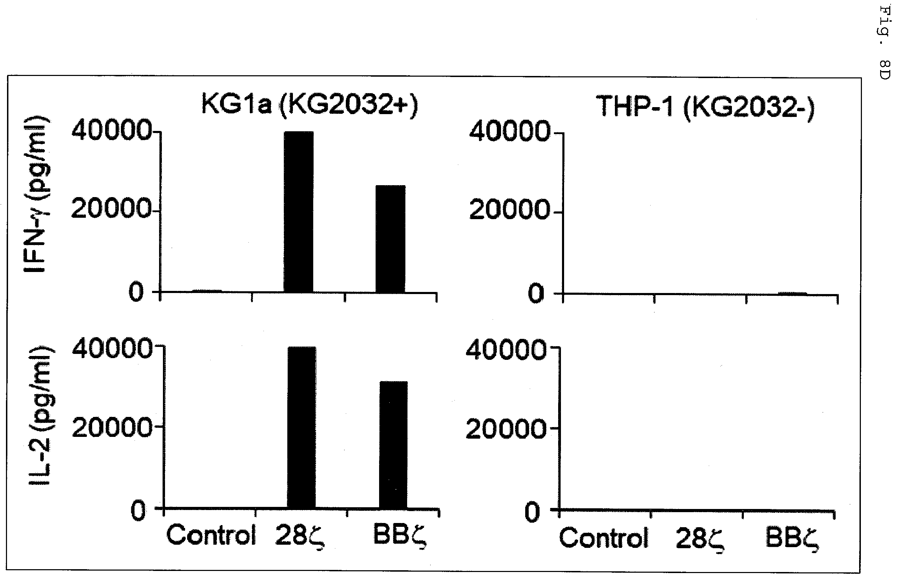

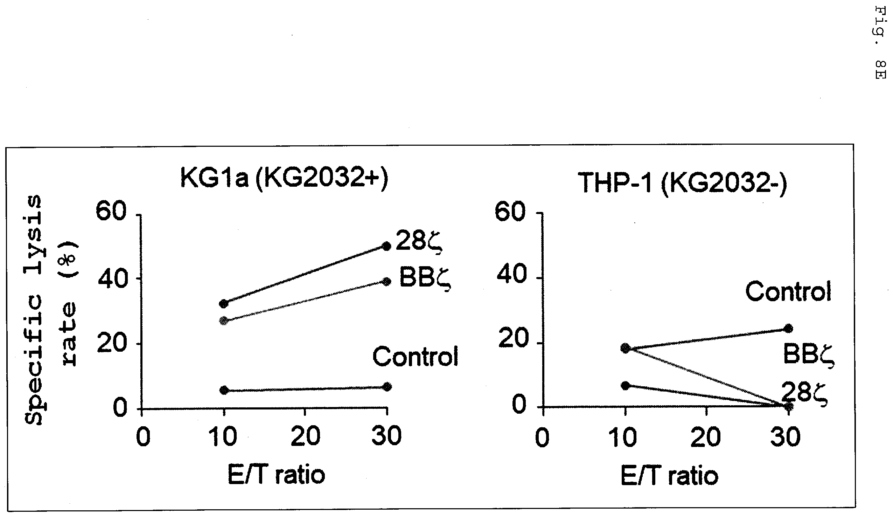

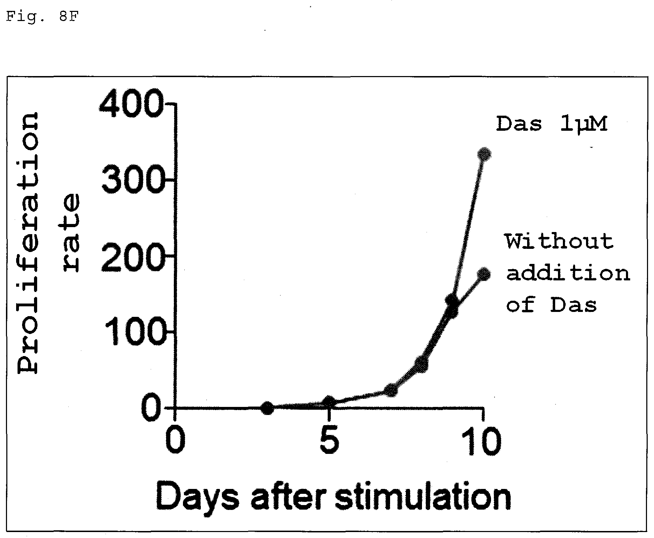

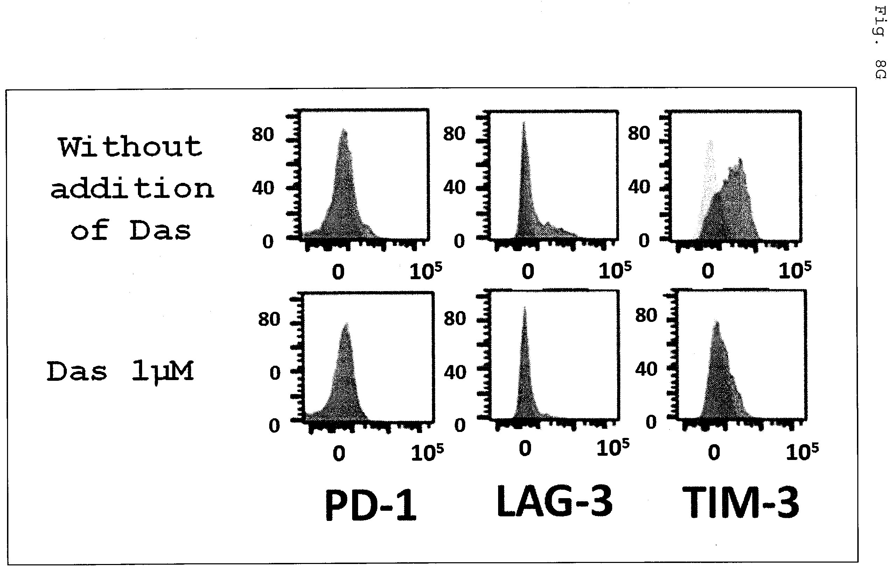

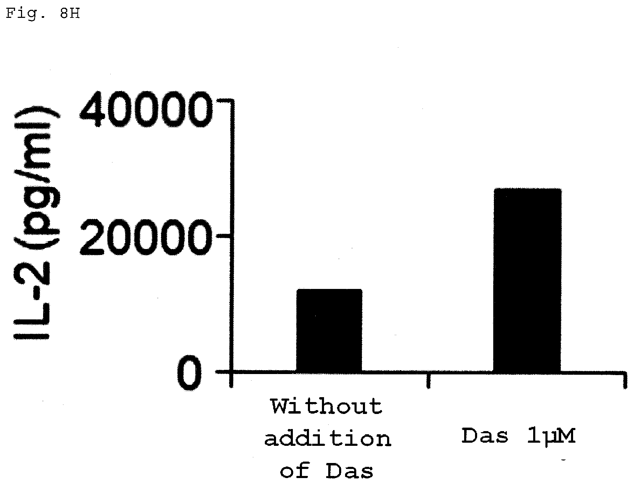

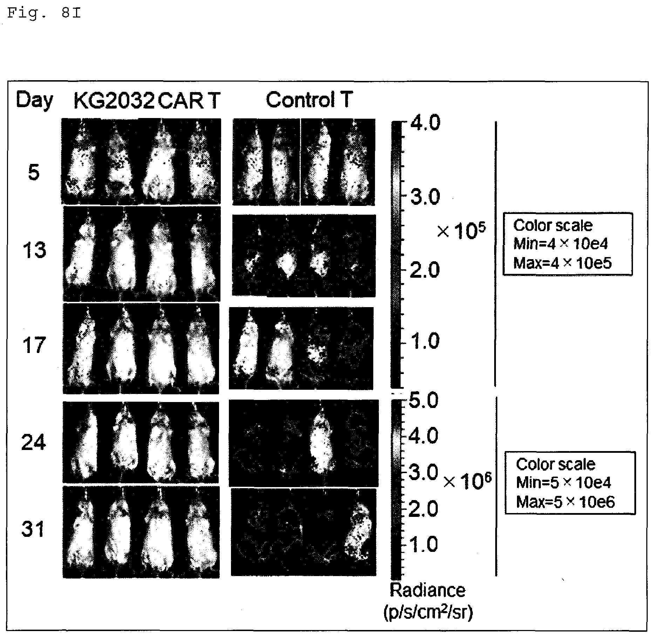

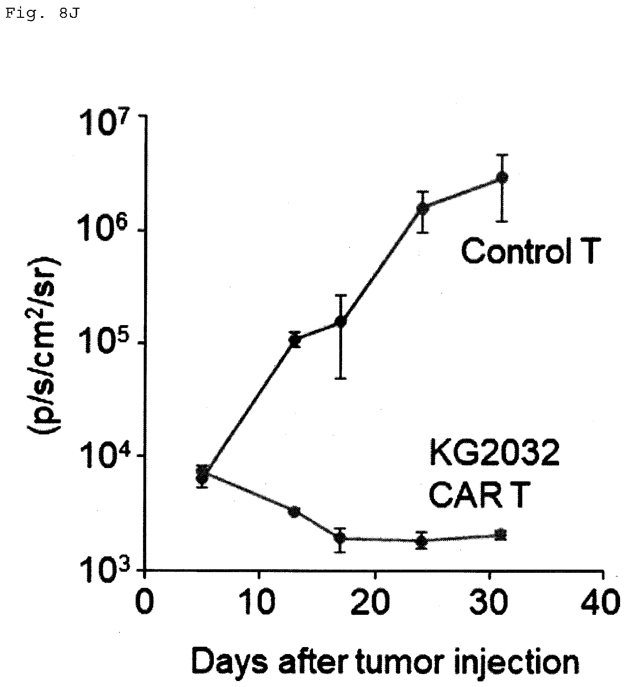

Subsequently, as a result of analyzing the sequences of gRNAs introduced into cells before sorting and into KG2032low cells after sorting using next-generation sequencing (NGS), HLA-DRA, HLA-DRB1, CIITA, and CD74 (FIG. 2C) were identified as molecules targeted by significantly enriched insert gRNAs in the KG2032low cells. Considering that the latter two are molecules necessary for HLA-DR to express on the cell surface, it was suggested that KG2032 recognized HLA-DR. Therefore, analyzing the binding of KG2032 to Daudi cells lacking HLA-DR, as shown in FIG. 2D, this binding was not observed, clearly indicating that the antigen recognized by KG2032 was HLA-DR. Furthermore, as shown in FIG. 2E, 8 clones are among the 32 clones and differ from KG2032, and it was also clarified that an antigen recognized by the 8 clones was HLA-DR.

The amino acid sequences of variable regions of these anti HLA-DR antibodies of the present invention are shown in Tables 1 and 2 below. The amino acid sequences of CDRs determined from the variable regions by Kabat are shown in Tables 3 and 4 below.

**[Table 1]**

| **clone No.** | **Heavy chain variable region (VH)** | |
|---|---|---|
| **KG2032** | QVQLQQSGPELVRPGVSVKISCKGSGYTFTD YAIHWFRQSHAKSLEWIGSISTYSGNPNYNQ NFKGKATMTVDKSSSTAYMELARLTSEDSA IYYCARYSELVFDYWGQGTTLTVSS | **(SEQ ID: 4)** |
| **KG19833** | EVQLQQSGPDLVKPGASVKISCKASGYSFTG YYMHWVKQSHGKSLEWIGRVNPDNGGTSY NQKFKGKAILTVDKSSSTAYMDLRSLTSEDS AVYYCARNHYYGYSDYGMDYWGQGTSVTV SS | **(SEQ ID: 37)** |
| **KG1982** | QVQLQQSGAVLARPGASVKLSCKASGYTFTS YWMQWVKQRPGQGLEWIGAIYPGDGDTRYT QKFKGKATLTADKSSSTAYMQLSSLTSEDS AVYYCAREGAYYVLFDYWGQGTTLTVSS | **(SEQ ID: 45)** |
| **aAML191872** | EVQLQQSGPELVKPGASVKISCRASGYTFTD YNIHWVKQSHGKSLEWIGYIYPYNGGTGYN QKFKSKATLTVNNSSSTAYMDLRSLTSEDS AVSYCAREARYPYGMDYWGQGTSVTVSS | **(SEQ ID: 53)** |
| **KG19130** | EVQLQQSGAELVKPGASVKLSCTASGFNIKD TYMHWVKQRPEQGLYWVGWIDPANGNTKH DPRFQDTATITADTTSNTAYLQLSSLTSEDT ALYYCARSLRYWCFDVWGAGTTVTVSS | **(SEQ ID: 61)** |
| **KG19122** | QVQLQQSGAELARPGASVKLSCKASGYTFTN HWMQWIKQRPGQGLEWIGAIYPGDGDTRYS QKFKGKATVTTDKSSSTAYMQLSSLASEDS AVYYCARRGFGYYDVMDYWGQGTSVTVSS | **(SEQ ID: 69)** |
| **aAML191870** | QVTLKESGPGILQSSQTLSLTCSFSGFSLSTS GIGVGWIRQPSGKGLEWLAHIWWNDNYYYN TALRSRLTISKDTSNNQVFLKIASVDAADTAT YYCARSSYFGNHLYYFDYWGQGTTLTVSS | **(SEQ ID: 73)** |
| **KG2053** | EVHLVESGGGLVKPGGSLKLSCAASGFIFSS YAMSWVRQTPEKRLEWVATISGGGTYIYYPD SVKGRFTISRDNAKNTLYLQMSSLRSEDTA MYYCARGENWFAYWGQGTLVTVSA | **(SEQ ID: 77)** |
| **KG19214** | QVQLQQSGAELARPGASVKLSCKASGYTFTN HWMQWIKQRPGQGLEWIGAIYPGDGDTRYS QKFKGKATVTTDKSSSTAYMQLSSLASEDS AVYYCARRGFGYYDVMDYWGQGTSVTVSS | **(SEQ ID: 69)** |

**[Table 2]**

| **clone No.** | **Light chain variable region (VL)** | |
|---|---|---|
| **KG2032** | DIKMTQSPSSMYASLGERVTITCKASQDIYS YLSWFQQKPGKSPKTLIYRANRLVDGVPSRF SGSGSGQDYSLTISSLEYEDLGIYYCLHNDE FPWTFGGGTKVEIK | **(SEQ ID: 8)** |
| **KG19833** | DIVMTQSPSSLSVSAGEKVTMSCKSSQSLLN SGNQKNYLAWYQQKPGQPPKLLIYGASTRES GVPDRFTGSGSGTDFTLTISSVQAEDLAVYY CQNDHSYPYTFGGGTKLEIK | **(SEQ ID: 41)** |
| **KG1982** | DIVLTQSPASLAVSLGQRATISCRASKSVSTS GYNYMHWYQQKPGQPPKLLIYLASNLESGV PARFSGSGSGTDFTLNIHPVEEEDAATYYCQ HSRELPWTFGGGTKLEIK DIQMTHSPASLSASVGETVTITCRASGNIHN YLAWYQQKQGKSPQLLVYNAKTLPDGVPSR FSGSGSGTQYSLKIYSLQTEDFGSYYCQHFW TTPYTFGGGTKLEIK | **(SEQ ID: 49)** |
| **aAML191872** | | **(SEQ ID: 57)** |
| **KG19130** | DIVMTQSHKFMSTLVGDRVSITCKASQDVS TTVAWYQQKPGQSPKLLIYSASYRYTGVPDR FTGSGSGTDFTFTISSVQAEDLAVYYCQQHY STPPTFGGGTKLEIK | **(SEQ ID: 65)** |
| **KG19122** | DIVMTQSHKFMSTSVGDRVSITCKASQDVS TTVAWYQQKPGQSPKLLIYSASYRYTGVPDR FTGSGSGTDFTFTISSVQAEDLAVYYCQQHY STPPTFGGGTKLEIK | **(SEQ ID: 65)** |
| **aAML191870** | DIVMTQSHKFMSTSVGDRVSITCKASQDVS TTVAWYQQKPGQSPKLLIYSASYRYTGVPDR FTGSGSGTDFTFTISSVQAEDLAVYYCQQHY STPPTFGGGTKLEIK | **(SEQ ID: 65)** |
| **KG2053** | DIVMTQSHKFMSTSVGDRVSITCKASQDVS TTVAWYQQKPGQSPKLLIYSASYRYTGVPDR FTGSGSGTDFTFTISSVQAEDLAVYYCQQHY STPPTFGGGTKLEIK | **(SEQ ID: 65)** |
| **KG19214** | DIVMTQSHKFMSTSVGDRVSITCKASQDVS TTVAWYQQKPGQSPKLLIYSVSYRYTGVPDR FTGSGSGTDFTFTISSVQAEDLAVYYCQQHY STPPTFGGGTKLEIK | **(SEQ ID: 79)** |

**[Table 1]**

| | **Heavy chain variable region (VH)** | | | | | |
|---|---|---|---|---|---|---|
| **clone No.** | **CDR1** | | **CDR2** | | **CDR3** | |
| **KGZ032** | **DYAIH** | **(SEQ ID: 1)** | **SISTYSGNPNYNQNFKG** | **(SEQ ID: 2)** | **YSELVFDY** | **(SEQ ID: 3)** |
| **KG19833** | **GYYMH** | **(SEQ ID: 34)** | **RVNPDNGGTSYNQKFKG** | **(SEQ ID: 35)** | **NHYYGYSDYGMDY** | **(SEQ ID: 36)** |
| **KG1982** | **SYWMQ** | **(SEQ ID: 42)** | **AIYPGDGDTRYTQKFKG** | **(SEQ ID: 43)** | **EGAYYVLFDY** | **(SEQ ID: 44)** |
| **aAML191872** | **DYNIH** | **(SEQ ID: 50)** | **YIYPYNGGTGYNQKFKS** | **(SEQ ID: 51)** | **EARYPYGMDY** | **(SEQ ID: 52)** |
| **KG19130** | **DTYMH** | **(SEQ ID: 58)** | **WIDPANGNTKHDPRFQD** | **(SEQ ID: 59)** | **SLRYWCFDV** | **(SEQ ID: 60)** |
| **KG19122** | **NHWMQ** | **(SEQ ID: 66)** | **AIYPGDGDTRYSQKFKG** | **(SEQ ID: 67)** | **RGFGYYDVMDY** | **(SEQ ID: 68)** |
| **aAML191870** | **TSGIGVG** | **(SEQ ID: 70)** | **HIWWNDNYYYNTALRS** | **(SEQ ID: 71)** | **SSYFGNHLYYFDY** | **(SEQ ID: 72)** |
| **KG2053** | **SYAMS** | **(SEQ ID: 74)** | **TISGGGTYIYYPDSVKG** | **(SEQ ID: 75)** | **GENWFAY** | **(SEQ ID: 76)** |
| **KG19214** | **NHWMQ** | **(SEQ ID: 66)** | **AIYPGDCDTRYSQKFKG** | **(SEQ ID: 67)** | **RGFGYYDVMDY** | **(SEQ ID: 68)** |

**[Table 4]**

| | **light chain variable region (VL)** | | | | | |
|---|---|---|---|---|---|---|
| **clone No.** | **CDR1** | | **CDR2** | | **CDR3** | |
| **KG2032** | **KASQDIYSYLS** | **(SEQ ID: 5)** | **RANRLVD** | **(SEQ ID: 6)** | **LHNDEFPWT** | **(SEQ ID: 7)** |
| **KG19833** | **KSSQSLLNSGNOQKNYLA** | **(SEQ ID: 38)** | **GASTRES** | **(SEQ ID: 39)** | **QNDHSYPYT** | **(SEQ ID: 40)** |
| **KG1982** | **RASKSVSTSGYNYMH** | **(SEQ ID: 46)** | **LASNLES** | **(SEQ ID: 47)** | **QHSRELPWT** | **(SEQ ID: 48)** |
| **aAML191872** | **RASGNIHNYLA** | **(SEQ ID: 54)** | **NAKTLPD** | **(SEQ ID: 55)** | **QHFWTTPYT** | **(SEQ ID: 56)** |
| **KG19130** | **KASQDVSTTVA** | **(SEQ ID: 62)** | **SASYRYT** | **(SEQ ID: 63)** | **QQHYSTPPT** | **(SEQ ID: 64)** |
| **KG19122** | **KASQDVSTTVA** | **(SEQ ID: 62)** | **SASYRYT** | **(SEQ ID: 63)** | **QQHYSTPPT** | **(SEQ ID: 64)** |
| **aAML191870** | **KASQDVSTTVA** | **(SEQ ID: 62)** | **SASYRYT** | **(SEQ ID: 63)** | **QQHYSTPPT** | **(SEQ ID: 64)** |
| **KG2053** | **KASQDVSTTVA** | **(SEQ ID: 62)** | **SASYRYT** | **(SEQ ID: 63)** | **QQHYSTPPT** | **(SEQ ID: 64)** |
| **KG19214** | **KASQDVSTTVA** | **(SEQ ID: 62)** | **SVSYRYT** | **(SEQ ID: 78)** | **QQHYSTPPT** | **(SEQ ID: 64)** |

### (Allele Specificity of HLA-DRB1 Recognized by KG2032)

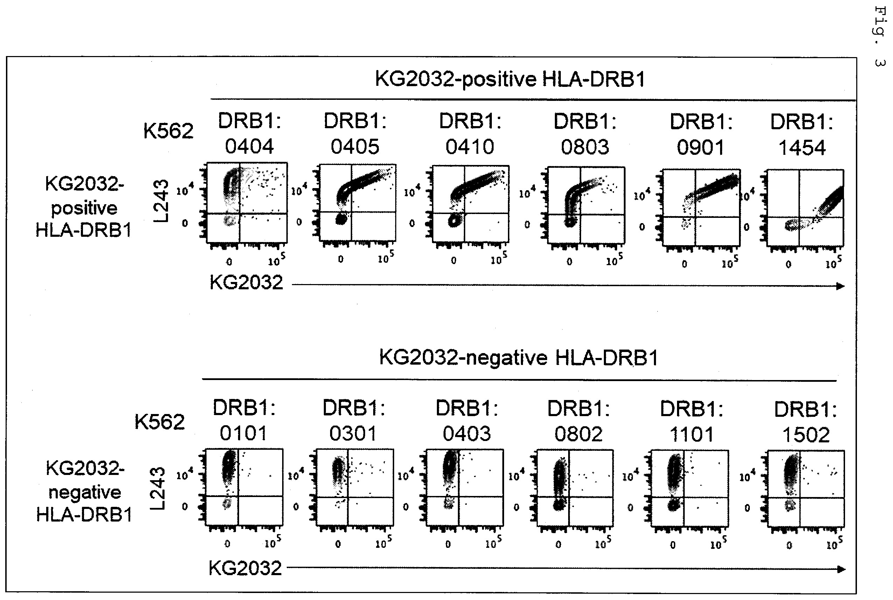

Due to the high polymorphism of HLA-DRB1, the present inventors investigated which alleles of HLA-DRB1 were recognized. As a result, as shown in FIG. 3, KG2032 bound to K562 cells expressing HLA-DRB1*0404, 0405, 0410, 0803, 0901, or 1454. On the other hand, KG2032 did not bind to K562 cells expressing HLA-DRB1*0101, 0403, 0802, 1101, or 1502.

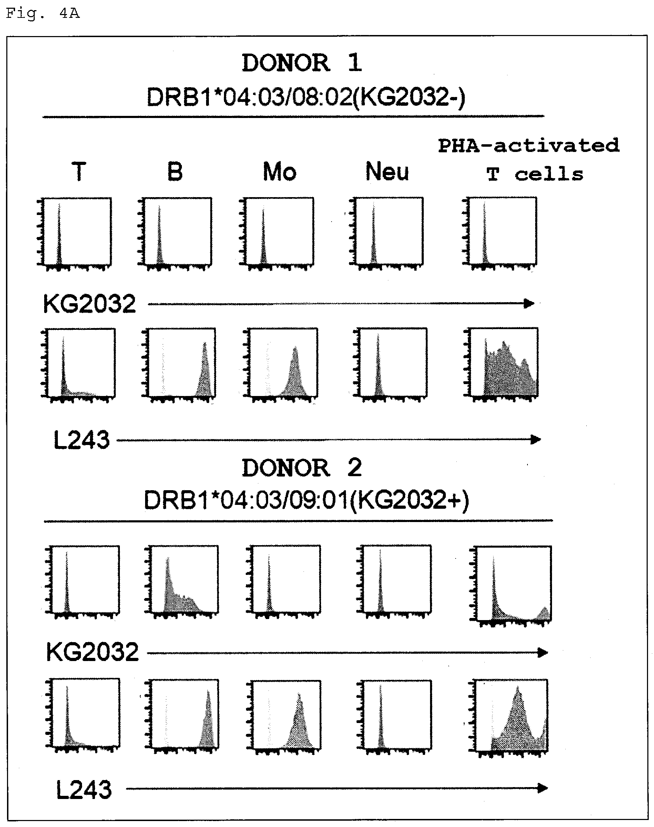

Furthermore, as shown in FIG. 4A, KG2032 did not bind to any cells of PBMCs from a donor with a KG2032-negative HLA-DRB1 gene (0403/0802). When peripheral blood from a donor with a KG2032-positive HLA-DRB1 gene, namely 0901, was stained, there was binding observed to B-cells and activated T-cells, but the binding was extremely weak compared to the existing anti-HLA-DR antibody L243.

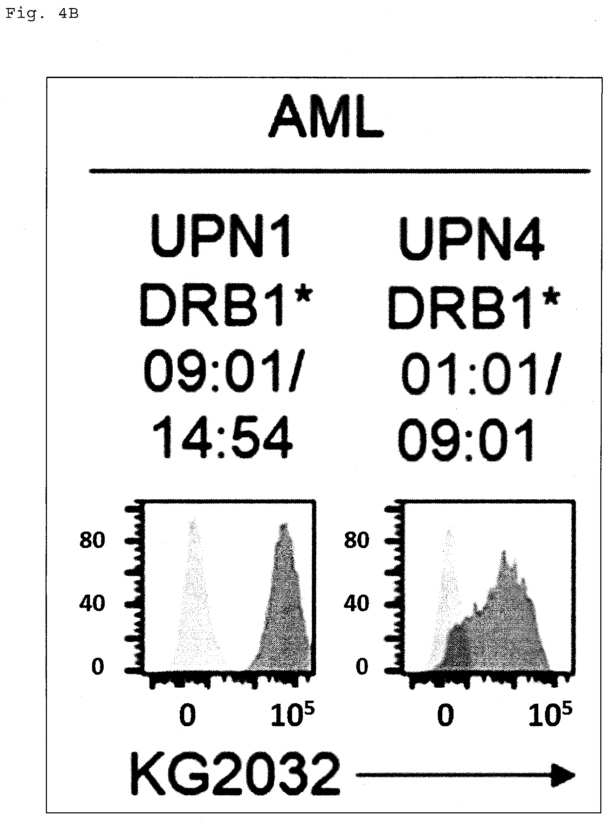

Additionally, as shown in FIG. 4A, KG2032 did not bind to monocytes or some T-cells that L243 binds to, namely those expressing HLA-DR. Also, as shown in FIG. 4B, binding of KG2032 to leukemia cells was observed in AML patients with a KG2032-positive HLA-DRB1 gene.

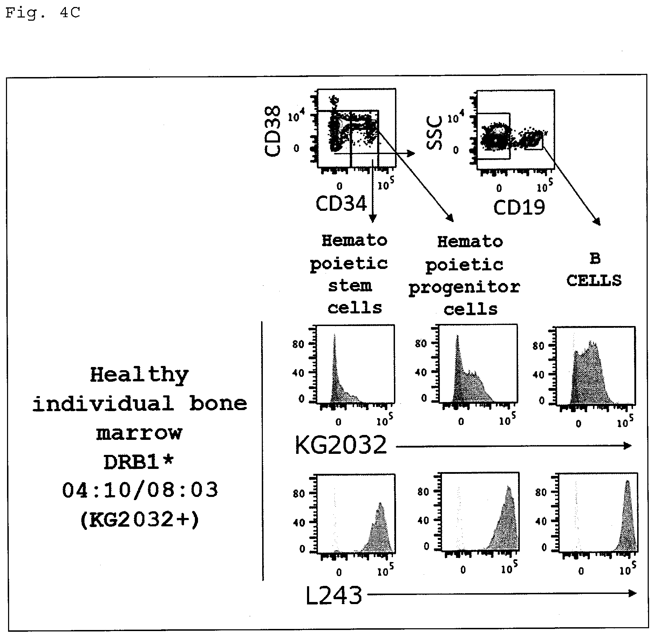

When bone marrow cells from donors with the KG2032-positive HLA-DRB1 genes 0410 and 0803 were stained, binding was observed to hematopoietic stem cells (CD34+CD38-) and hematopoietic progenitor cells (CD34+CD38+), but the binding was extremely weak compared to the existing anti-HLA-DR antibody L243. Furthermore, as shown in FIG. 4C, KG2032 only bound to a very small subset of hematopoietic stem cells that L243 binds to, namely those expressing HLA-DR.

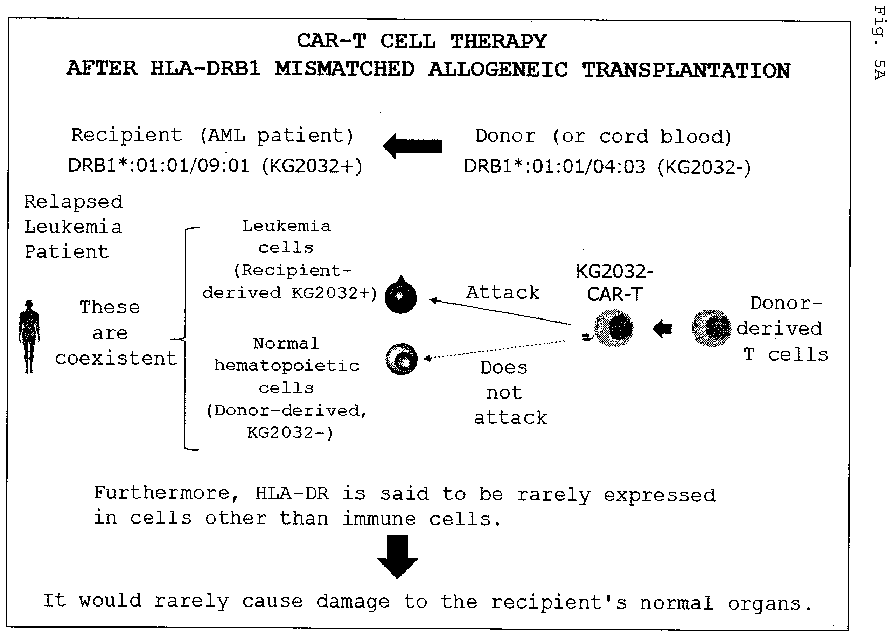

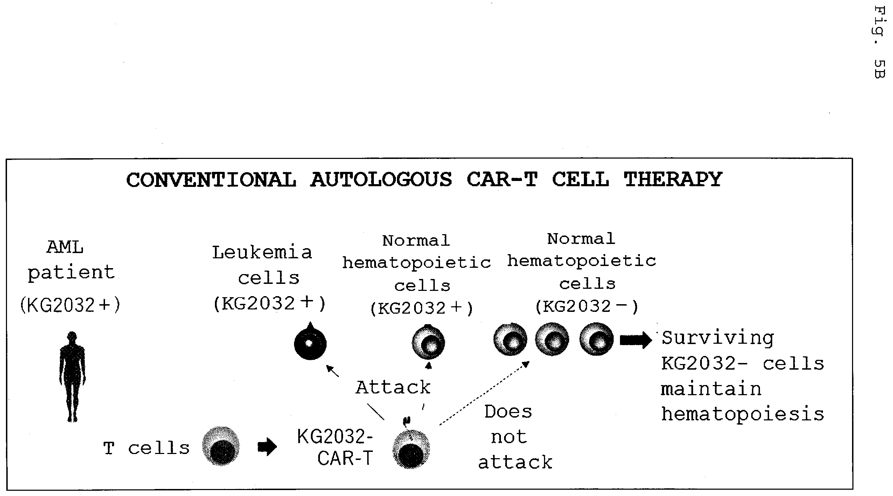

From these results, the following example applications of KG2032 in AML treatment can be considered.
(1) In AML patients with relapse after HLA-DR mismatched allogeneic transplantation, if the HLA-DR of the recipient (leukemia patient) is of the KG2032-positive type and the HLA-DR type of the donor is of the KG2032-negative type, KG2032-derived CAR-T cells created from the transplant patient themselves or donor-derived T cells would specifically attack the leukemia of the recipient (FIG. 5A).
(2) KG2032 only binds to certain cells such as B cells in normal blood cells, and it does not bind to all the hematopoietic stem cells serving as the source of all blood cells. Therefore, even if conventional autologous CAR-T cells are used for treatment in AML patients with the KG2032-positive HLA-DRB1 type, leukemia cells would be eliminated, and normal hematopoiesis would be maintained by KG2032-negative cells. Thus, it is believed to be viable as a treatment (FIG. 5B).

### (Identification of Epitope Recognized by KG2032)

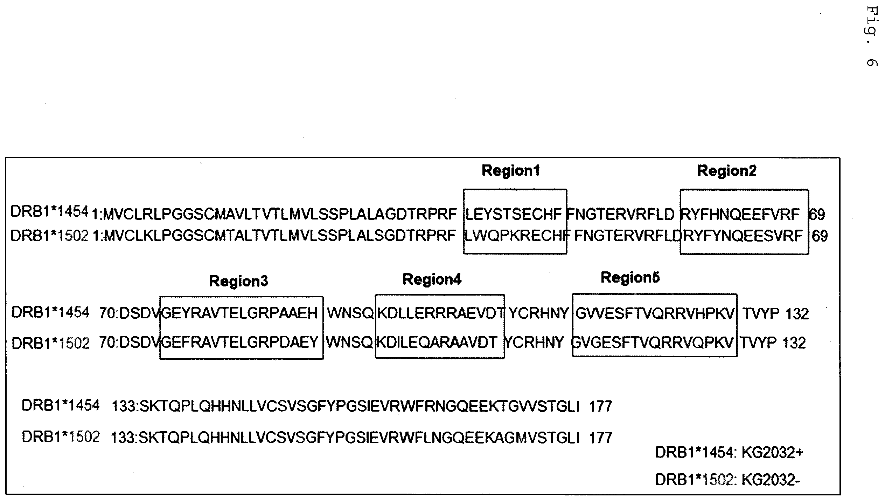

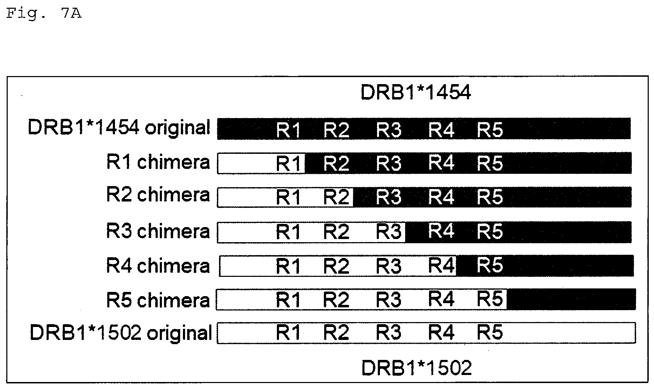

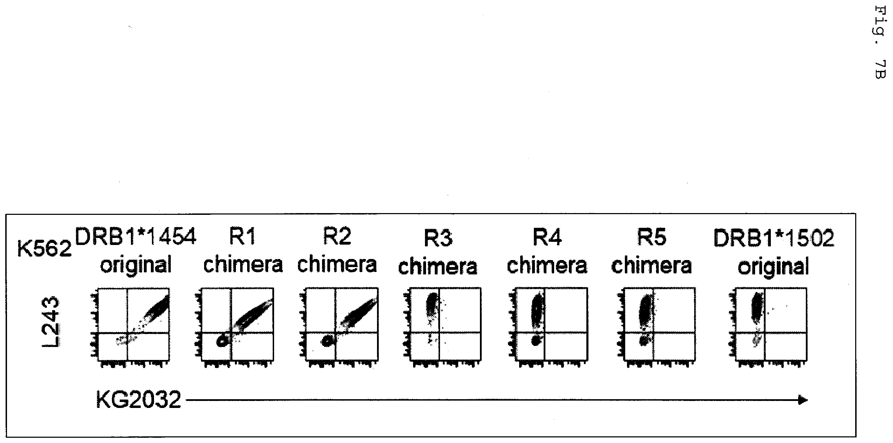

In order to identify the epitope recognized by KG2032, the amino acid sequences of HLA-DRB1*1454 (KG2032-positive) and HLA-DRB1*1502 (KG2032-negative) were compared (FIG. 6). Furthermore, as shown in FIG. 7A, an expression vector expressing the chimeric protein of HLA-DRB1*1454 and HLA-DRB1*1502 was created using overlapping PCR. These were then introduced into K562 cells by retrovirus, and stained with KG2032 and L243 two days later to analyze the presence or absence of binding by flow cytometry. As a result, as shown in FIG. 7B, Region3 (A.A. 74-89) shown in FIG. 6 was shown to be essential for the binding of KG2032.

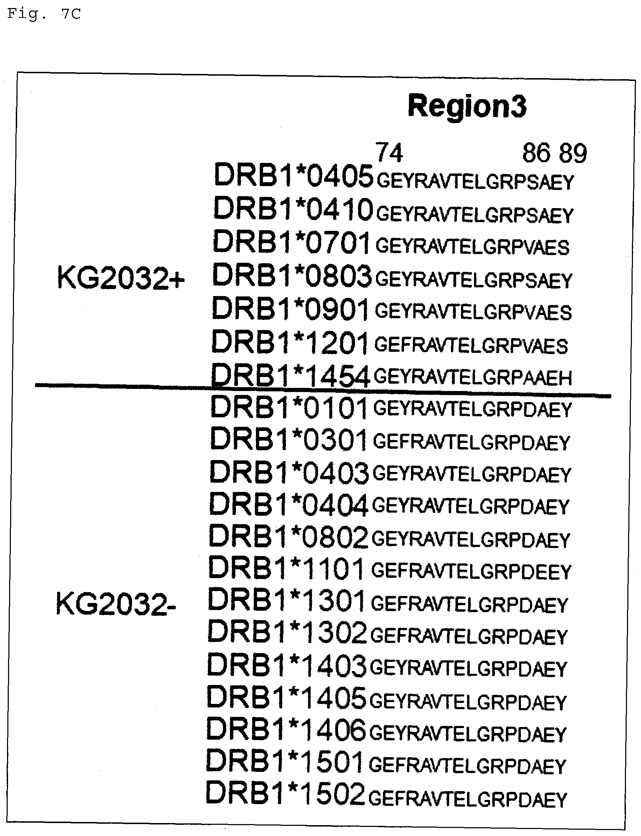

Furthermore, upon comparing the amino acid sequences of Region3 of the HLA-DRB1 that binds with KG2032 and the HLA-DRB1 that does not, as shown in FIG. 7C, it was found that the amino acid at position 86 was always aspartic acid (D) in the HLA-DR that does not bind with KG2032, whereas it was serine (S), valine (V), or alanine (A) in the HLA-DR that does.

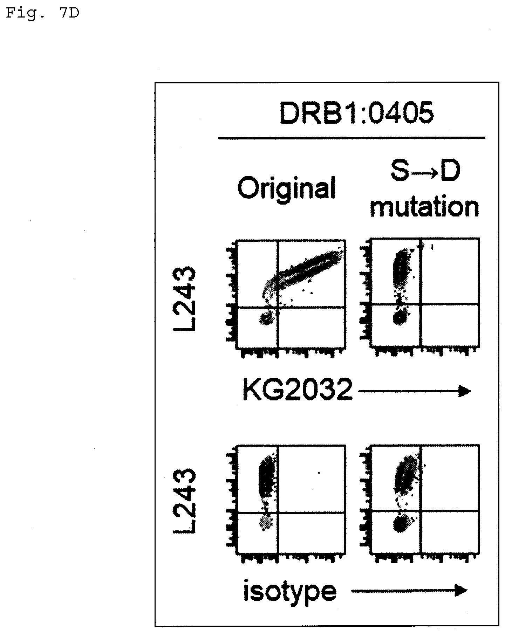

Therefore, the serine (S) at position 86 of HLA-DRB1*0405, which binds with KG2032, was substituted with aspartic acid (D) and expressed in K562 cells. As a result, it was revealed that the binding of L243 was retained, but the binding of KG2032 was lost, as shown in FIG. 7D.

From these findings, it has been shown that for KG2032 to bind, the amino acid at position 86 of Region3 must be an amino acid other than aspartic acid (such as serine, valine, or alanine).

### (Development of CAR-T Cells Derived from KG2032 NonReactive DR Donor)

A chimeric antigen receptor containing the variable region of KG2032 (KG2032-CAR) was prepared. First, the cDNA of the variable part of KG2032 was obtained by the 5' RACE method using the SMARTer RACE 5'/3' Kit (Takara), and its sequence was identified. Then, as shown in FIG. 8A, the light chain (VL) and heavy chain (VH) were fused using overlapping PCR, with a linker sequence in between. After that, this, along with CD28 and CD3ζ, or CD8α, 4-1BB, and CD3ζ, was fused by overlapping PCR, creating two types of KG2032-CAR (KG2032-CD28-CD3ζ-CAR and KG2032-BBz-CAR) . The configuration of these CARs is shown in Table 5 below.

**[Table 5]**

| | **Signal Peptide** | **Antigen Recognition Site (s c F v)** | | | **Transmembrane Domain** | **Intracellular Signaling Domain** | |
|---|---|---|---|---|---|---|---|
| **KG2032-CD28-CD3ζ-CAR (SEQ ID : 9)** | **IgK (Positions 1 - 22)** | **KG2032 Light Chain Variable Region (Positions 23 - 130)** | **Whitlow /218 linker (Positions 131 - 145)** | **KG2032 Heavy Chain Variable Region (Positions 146 - 262)** | **CD28 (Positions 266 - 331)** | **CD28 (Positions 332 - 372)** | **CD3ζ (Positions 373 - 484)** |
| **KG2032-BBz - CAR (SEQ ID : 10)** | **IgK (Positions 1 - 22)** | **KG2032 Light Chain Variable Region (Positions 23 - 130)** | **Whitlow /2 1 8 linker (Positions 131 - 145)** | **KG2032 Heavy Chain Variable Region (Positions 146 - 262)** | **CD8α (Positions 263 - 331)** | **4 - 1BB (Positions 332 - 373)** | **CD3ζ (Positions 374 - 485)** |
| 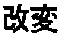 **KG2032 CAR (SEQ ID : 3 3)** | **IgKVIII (Positions 1-19)** | **KG2032 Heavy Chain Variable Region (Positions 20 - 136)** | **Whitlow /2 1 8 linker (Positions 137 - 151)** | **KG2032 Light Chain Variable Region (Positions 152 - 259)** | **CD8α (Positions 260-345)** | **4 - 1BB (Positions 346 - 387)** | **CD3ζ (Positions 388-499)** |

Next, KG2032 CAR was introduced into T lymphocytes derived from a KG2032-negative HLA-DRB1 (0403/0802) donor using a retroviral vector, creating KG2032-CAR-T cells (FIGs. 8B and 8C).

The CAR-T cells thus prepared, when cocultured with KGla cells, to which KG2032 binds, produced IFN-γ and IL-2, and exhibited cytotoxic activity, as shown in FIGs. 8D and 8E. On the other hand, when cocultured with THP-1 cells, to which KG2032 does not bind, no cytokine production or cytotoxic activity was observed.

Furthermore, these results were observed whether the costimulatory molecule was CD28 or 4-1BB, so that in the following experiments, only KG2032-CAR (KG2032-BBz-CAR), where 4-1BB is the costimulatory molecule, was evaluated.

Next, in order to prevent exhaustion of the CAR-T cells, an attempt was made to add dasatinib, a tyrosine kinase inhibitor, during the production of the CAR-T cells. As a result, as shown in FIGs. 8F to H, treatment with 1 µM dasatinib promoted the proliferation of KG2032-BBz-CAR-T cells, while the expression of exhaustion marker molecules (PD-1, TIM-3, and LAG-3) decreased. Also, IL-2 cytokine production during cocultivation with KGla increased.

Next, after transplanting KGla cells expressing luciferase into NOG mice, KG2032-BBz CAR-T cells were administered, and the anti-leukemia effect thereof was evaluated. As a result, as shown in FIGs. 8I and J, a prominent anti-leukemia effect was observed.

### (Development of CAR-NK Cells Derived from KG2032 NonReactive DR Donor)

A vector for co-expressing the chimeric antigen receptor fused to the variable region of KG2032 with CD28 and CD3ζ and IL-15 was created. Then, NK cells expressing this chimeric antigen receptor (CAR-NK cells) were prepared and cocultured with a cell line derived from human acute myeloid leukemia (KGla cells). As a result, although not shown in the figures, the CAR-NK cells were also found to exhibit cytotoxic activity. Moreover, it was revealed that when the CAR-NK cells were administered to mice transplanted with KGla cells, a prominent antitumor effect was observed as well.

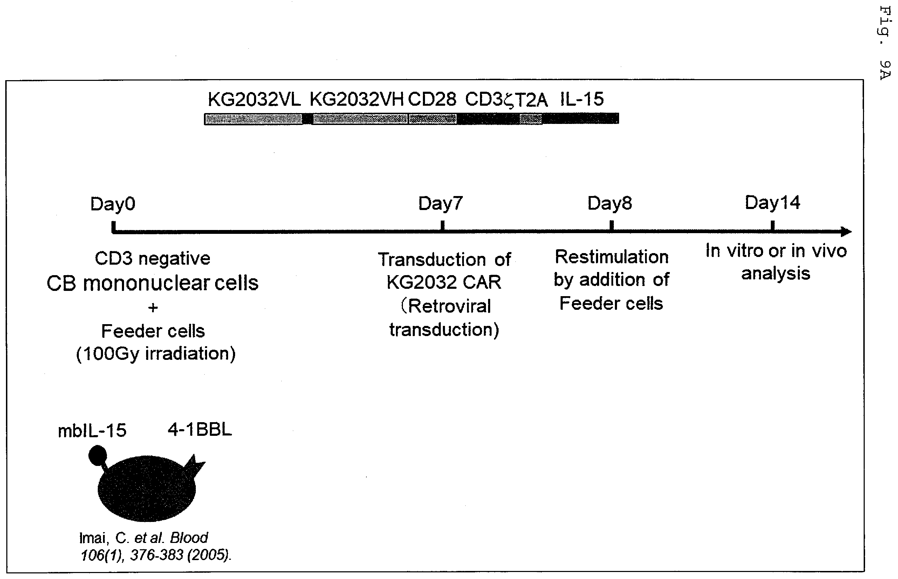

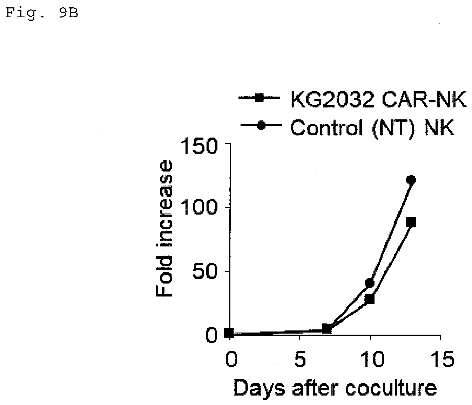

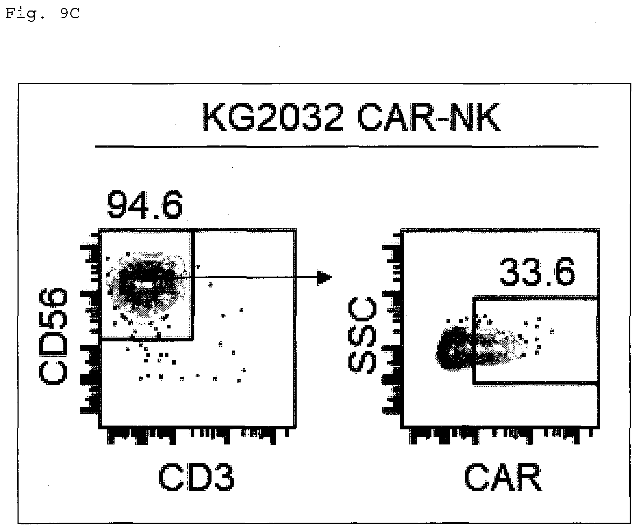

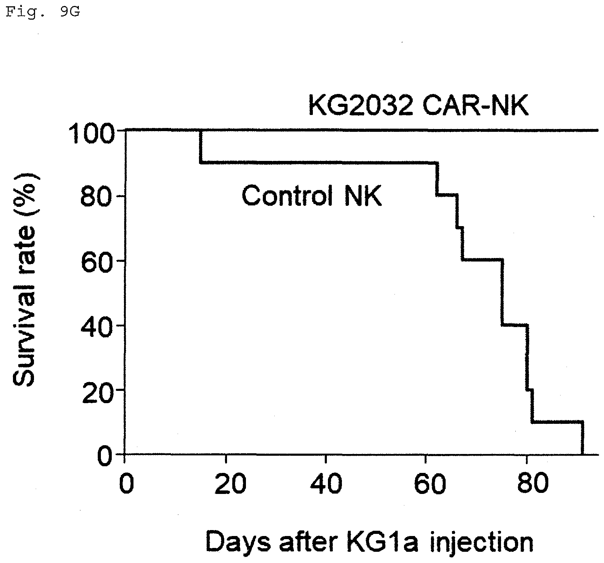

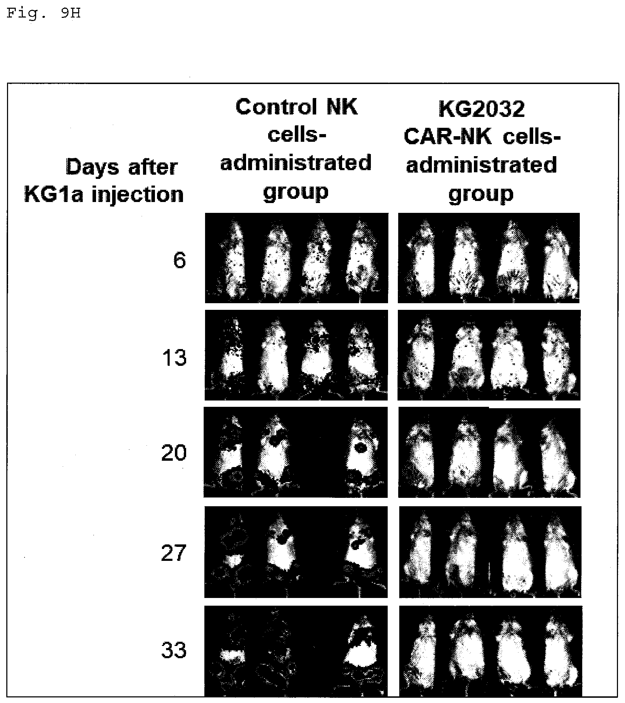

To explain specifically, the present inventors generated KG2032 CAR constructs for CAR-NK cells by fusing the variable regions of KG2032, CD28, and CD3ζ. In addition, the present inventors designed a retroviral vector to co-express IL-15 together with KG2032 CAR to enhance the proliferation and survival of CAR-NK cells. The present inventors expanded NK cells from T-cell-depleted human CB cells carrying the KG2032-non-reactive HLA-DRB1 through stimulation with irradiated K562 cells expressing 4-1BB ligand and membrane-bound IL-15. Fourteen days after the start of the culture, the present inventors analyzed CB-derived NK cells that had been retrovirally transduced with KG2032 CAR/IL-15 (Figs.9A-9C).

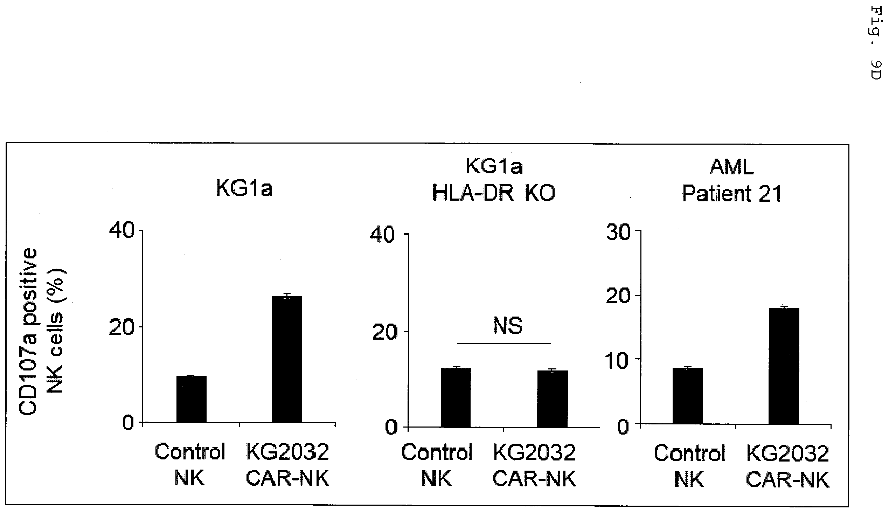

CD107a degranulation was significantly increased in KG2032 CAR-NK cells compared with non-transduced NK cells upon co-culture with KGla cells or patient-derived primary AML cells, but not with HLA-DRB1-deficient KGla cells (Fig.9D).

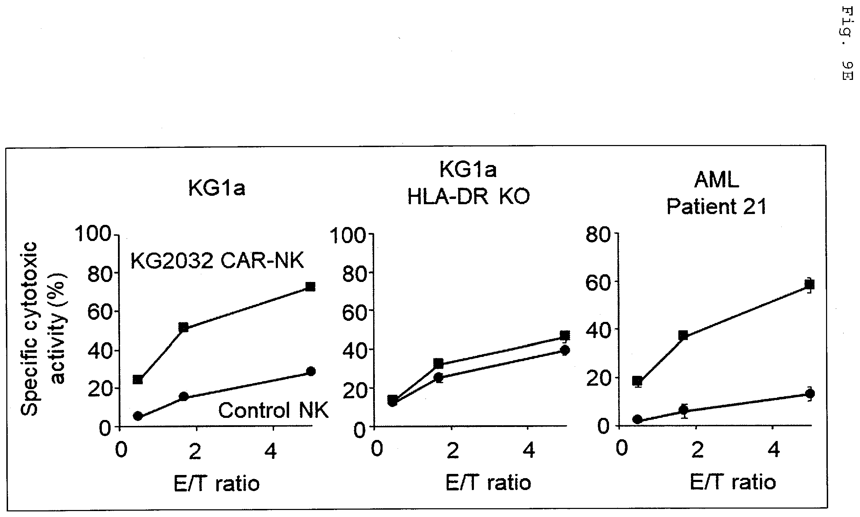

KG2032 CAR-NK cells exerted significant cytotoxic activity against KGla cells and patient-derived primary AML cells, but not against HLA-DRB1-deficient KGla cells (Fig.9E).

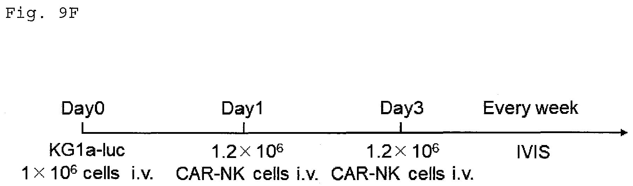

Furthermore, KG2032 CAR-NK cells significantly reduced the leukemia burden and extended survival in the aforementioned mouse xenograft model based on KGla AML cell transplantation (Figs.9F-9H).

### (Development of Modified KG2032 CAT-T cells)

Severe cytokine release syndrome (CRS) may enhance HLA-DR expression in non-hematological tissues. Therefore, severe CRS should be avoided to maximize the safety of KG2032 CAR-T cells. It has been reported that modifying CAR constructs can prevent severe CRS without losing the efficacy of CAR-T cells (Ying,Z. et al.A safe and potent anti-CD19 CAR-T cell therapy. Nat Med 25, 947-953 (2019). https://doi.org:10.1038/s41591-019-0421-7). Therefore, in order to reduce cytokine production by CAR-T cells during co-culture with AML cells, the present inventors changed the order of the heavy chain (VH) and light chain (VL) in the antigen recognition domain, extended the length of the CD8α hinge/transmembrane domain, and thus the KG2032 CAR construct was modified. Furthermore, to further attenuate CAR activation signal, mutations were introduced in two of the three immunoreceptor tyrosine-based activation motif (ITAM) domains of the CD3ζ sequence (Feucht,J. et al.Calibration of CAR activation potential directs alternative T cell fates and therapeutic potency.Nat Med 25,82-88(2019)). Note that, the structure of this modified KG2032 CAR is shown in Table 5 above.

At first, the present inventors analyzed the secretion of IFN-γ by the original KG2032 CAR-T cells or the modified KG2032 CAR-T cells after co-culture with KGla AML cells. As a result, the secretion of IFN-γ by the modified KG2032 CAR-T cells was reduced compared to that of the original, and it was confirmed that the mutations weakened the CAR activation signal.

Next, the present inventors analyzed the secretion levels of IFN-γ and IL-2 by the modified KG2032 CAR-T cells after co-culture with normal intestinal epithelial cells purified from unaffected small bowels of patients with colon cancer or KGla cells. As a result, the modified KG2032 CAR-T cells were not activated by co-culture with normal intestinal epithelial cells carrying the KG2032-reactive HLA-DRB1. On the other hand, the modified KG2032 CAR-T cells retained cytotoxic activity against KGla AML cells and patient-derived primary AML cells.

In addition, the modified KG2032 CAR-T cells were administered to mice transplanted with KGla AML cells. As a result, administration of the modified KG2032 CAR-T cells significantly decreased the number of KGla AML cells engrafted in the mice and prolonged their survival period. Note that, no obvious toxicity was observed in the mice treated with the modified KG2032 CAR-T cells.

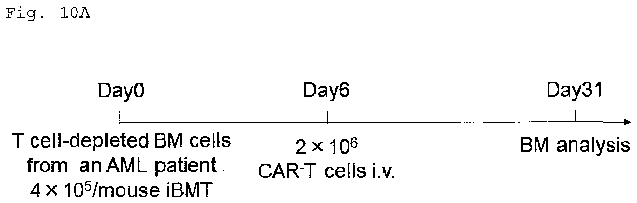

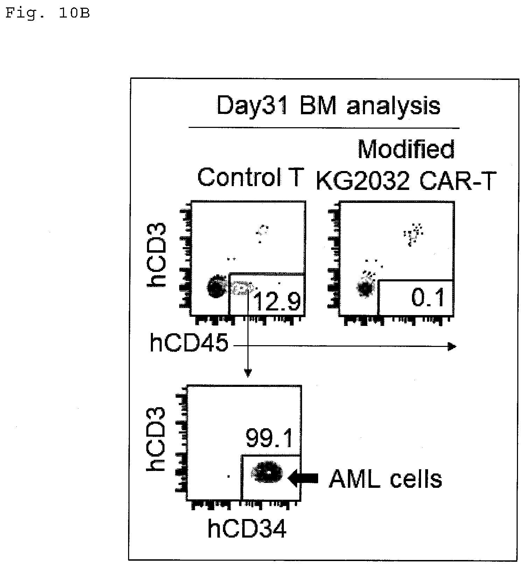

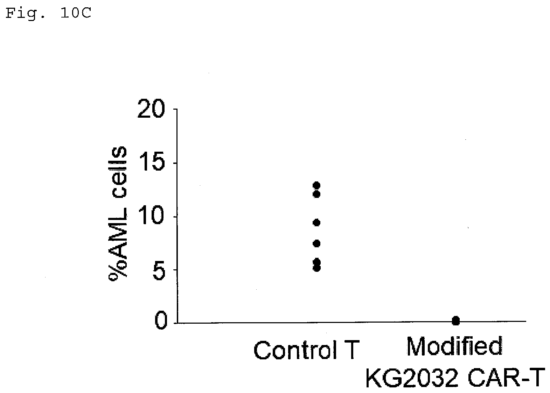

Furthermore, the modified KG2032 CAR-T cells were administered to immunodeficient mice transplanted with patient-derived AML cells (Fig.10A). As a result, the modified KG2032 CAR-T cells eradicated primary AML cells engrafted in the immunodeficient mice (Figs.10B and 10C). Note that, no obvious toxicity was observed in the mice administered with the modified KG2032 CAR-T cells.

### [Industrial Applicability]

As described above, according to the present invention, by targeting HLA-DR, which is a cell surface antigen specific to blood cancers such as acute myeloid leukemia, it becomes possible to treat the disease using antibodies that recognize this antigen or functional fragments thereof, or using a chimeric antigen receptor or the like that has it.

In particular, such antibodies can show allele specificity to HLA-DR. For blood cancer patients with relapse after HLA-DR mismatched allogeneic transplantation, if the HLA-DR of the recipient (blood cancer patient) is a positive type that the antibody of the present invention binds to, and the HLA-DR type of the donor is a negative type that the antibody of the present invention does not bind to, then CAR-T cells derived from the antibody of the present invention created from donor-derived cells can specifically attack the blood cancer cells of the recipient.

Furthermore, if the antibody of the present invention binds only to some cells, such as B cells, in normal blood cells and does not bind to monocytes and the like, even if regular autologous CAR-T cells and the like are used for treatment in blood cancer patients with the positive HLA-DR that the antibody of the present invention binds to, blood cancer cells are eliminated, normal hematopoiesis is maintained by negative cells that the antibody of the present invention does not bind to, making treatment possible.

Therefore, the present invention is useful in therapeutic agents for a blood cancer and development thereof, and the like.

## Claims

1. A composition for treating a blood cancer, comprising: an antibody that recognizes HLA-DR or a functional fragment thereof.

2. The composition according to claim 1, comprising: an immune cell expressing a chimeric antigen receptor that has the antibody or a functional fragment thereof.

3. The composition according to claim 1, comprising: a multispecific antibody that has the antibody or a functional fragment thereof and an antigen recognition site for an immune cell.

4. The composition according to claim 1, wherein the antibody or a functional fragment thereof does not bind to monocytes.

5. The composition according to claim 4, comprising: an immune cell expressing a chimeric antigen receptor that has the antibody or a functional fragment thereof.

6. The composition according to claim 4, comprising: a multispecific antibody that has the antibody or a functional fragment thereof and an antigen recognition site for an immune cell.

7. The composition according to claim 1, wherein the antibody or a functional fragment thereof binds to an allele type of HLA-DRB1 in which position 86 is an amino acid other than aspartic acid, but does not bind to an allele type of HLA-DRB1 where position 86 is aspartic acid.

8. The composition according to claim 1, wherein the antibody
binds to a region containing an amino acid sequence from positions 74 to 89 of HLA-DRB1, where an amino acid at position 86 is an amino acid other than aspartic acid, but
does not bind to a region containing the amino acid sequence from positions 74 to 89 of HLA-DRB1, where the amino acid at position 86 is aspartic acid.

9. An antibody that recognizes HLA-DR or a functional fragment thereof, which has a feature described in one of following (a) to (i):
(a) comprising
a heavy chain variable region that includes complementarity-determining regions 1 to 3 containing amino acid sequences set forth in SEQ ID NOs: 1 to 3, respectively,
a light chain variable region that includes complementarity-determining regions 1 to 3 containing amino acid sequences set forth in SEQ ID NOs: 5 to 7, respectively, ;
(b) comprising a heavy chain variable region that includes complementarity-determining regions 1 to 3 containing amino acid sequences set forth in SEQ ID NOs: 34 to 36, respectively, and
a light chain variable region that includes complementarity-determining regions 1 to 3 containing amino acid sequences set forth in SEQ ID NOs: 38 to 40, respectively;
(c) comprising
a heavy chain variable region that includes complementarity-determining regions 1 to 3 containing amino acid sequences set forth in SEQ ID NOs: 42 to 44, respectively, and
a light chain variable region that includes complementarity-determining regions 1 to 3 containing amino acid sequences set forth in SEQ ID NOs: 46 to 48, respectively;
(d) comprising
a heavy chain variable region that includes complementarity-determining regions 1 to 3 containing amino acid sequences set forth in SEQ ID NOs: 50 to 52, respectively, and
a light chain variable region that includes complementarity-determining regions 1 to 3 containing amino acid sequences set forth in SEQ ID NOs: 54 to 56, respectively;
(e) comprising
a heavy chain variable region that includes complementarity-determining regions 1 to 3 containing amino acid sequences set forth in SEQ ID NOs: 58 to 60, respectively, and
a light chain variable region that includes complementarity-determining regions 1 to 3 containing amino acid sequences set forth in SEQ ID NOs: 62 to 64, respectively;
(f) comprising
a heavy chain variable region that includes complementarity-determining regions 1 to 3 containing amino acid sequences set forth in SEQ ID NOs: 66 to 68, respectively, and
a light chain variable region that includes complementarity-determining regions 1 to 3 containing amino acid sequences set forth in SEQ ID NOs: 62 to 64, respectively;
(g) comprising
a heavy chain variable region that includes complementarity-determining regions 1 to 3 containing amino acid sequences set forth in SEQ ID NOs: 70 to 72, respectively, and
a light chain variable region that includes complementarity-determining regions 1 to 3 containing amino acid sequences set forth in SEQ ID NOs: 62 to 64, respectively;
(h) comprising
a heavy chain variable region that includes complementarity-determining regions 1 to 3 containing amino acid sequences set forth in SEQ ID NOs: 74 to 76, respectively, and
a light chain variable region that includes complementarity-determining regions 1 to 3 containing amino acid sequences set forth in SEQ ID NOs: 62 to 64, respectively; and
(i) comprising
a heavy chain variable region that includes complementarity-determining regions 1 to 3 containing amino acid sequences set forth in SEQ ID NOs: 66 to 68, respectively, and
a light chain variable region that includes complementarity-determining regions 1 to 3 containing amino acid sequences set forth in SEQ ID NOs: 62, 78 and 64, respectively.

10. An immune cell expressing a chimeric antigen receptor that has the antibody or a functional fragment thereof according to claim 8 or 9.

11. A pharmaceutical composition comprising: an immune cell expressing the antibody or a functional fragment thereof according to claim 8 or 9, or a chimeric antigen receptor including the antibody or a functional fragment thereof.

12. A composition to be administered to a blood cancer patient transplanted with hematopoietic stem cells,
wherein the composition comprises an antibody that recognizes HLA-DR or a functional fragment thereof,
a donor of the hematopoietic stem cells and the patient have different HLA-DR allele types, and
the antibody or a functional fragment thereof binds to HLA-DR of the patient but does not bind to HLA-DR of the donor.
